# EUROPEAN PATENT APPLICATION

(11) **EP 4 303 308 A1**
(43) Date of publication of application: **10.01.2024**
(21) Application number: 22306019.5
(22) Date of filing: 07.07.2022
(51) Int. Cl.: C12P 7/26

(54) **RECOMBINANT HOST CELLS PRODUCING IRONES AND USES THEREOF**

(71) Applicant: MUSEUM NATIONAL D'HISTOIRE NATURELLE, 75005 Paris (FR); Centre national de la recherche scientifique, 75016 Paris (FR); Sayous, Victor, 75004 Paris (FR)
(72) Inventor: SAYOUS, Victor, 75004 Paris (FR); LI, Yanyan, 75005 Paris (FR)
(74) Representative: Cabinet Becker et Associés

(57) **Abstract**

The present invention relates to a recombinant host cell genetically modified to produce irone compounds. It also relates to a method for producing irone compounds 10 using said recombinant host cell.

## Description

### FIELD OF THE INVENTION

The present invention relates to the field of the production of biobased compounds, in particular the production of irone compounds using recombinant host cells.

### BACKGROUND OF THE INVENTION

Plant natural products (PNPs) are non-nutritive, biologically active compounds that are widely used as drugs, chemicals, flavors and fragrances. These compounds are extracted from plant biomass, whose supply require substantial investment in land, water, and time and are often limited. Moreover, PNPs are frequently present in the plants with low abundance, which makes them unable to meet the research and market demand. Some PNPs can be chemically synthetized, however, it is generally a challenge to obtain structurally complex, enantiopure bioactive PNPs by synthesis at large scale.

Recent development in synthetic biology and metabolic engineering has allowed the production of PNPs in fast growing and fermentable microorganisms. This strategy is one of the most promising alternatives to access high-value molecules, by circumventing the issue of limited and inconsistent supply of plant biomass. To do this, complete or partial plant biosynthetic pathways are transferred into an engineered microbial chassis, such as *Saccharomyces cerevisiae* or *Escherichia coli,* enabling heterologous production of final PNPs or their biosynthetic intermediates for downstream chemical synthesis. Successful examples using this approach include the production of semi-synthetic artemisinin (Paddon et al., Nat. Rev. Microbiol. 12 (2014) 355-367), rose oil (Kutyna et al., Genes 2018, 9(7), 326) and resveratrol (Li et al., Scientific Reports, 2016, volume 6, Article number: 36827). However, in many cases, the native biosynthetic pathways in plants remain completely or partially unknown. Thus, de novo design of a new pathway or using alternative enzymes for missing steps is required for heterologous production.

The orris oil is one of the most expensive natural products used in perfumery, costing more than 140,000 €/kg. Despite its high price, the production worldwide of orris oil is decreasing, due to a long production cycle and low yields which is not incentive enough for farmers. Irones are the active compounds of the orris oil, responsible for its violet-like scent with powdery notes. Five stereoisomers/enantiomers of irones are naturally found in fresh orris extracts with different proportions. The cis-α-, β- and γ-irones are present in most cases, whereas trans-α- and γ-irones are rare. The smell and the price of the orris oil are determined by the ratio between different irones

Chemical synthesis of single enantiomer of irone is challenging due to the two chiral centers; and there exist few reports. The commercial Irone Alpha^{®} is composed of mainly diastereoisomeres of α-irone in presence of a minor quantity of β-irone. The synthesis involves an acid-catalyzed cyclization of methyl-3-peusdo ionone. Starting from the above-mentioned Irone Alpha^{®}, a biocatalyst-assisted procedure affording all ten isomers of irones has been developed (Brenna et al. Helvetica Chimica Acta , 2001, 84(12):3650-3666). However, this process is long and requires several lipase-mediated resolutions of racemic mixtures and separation of diastereomeric products and is not adapted for industrial production. A recent study reported challenging ex novo enantioselective synthesis of (-)-(2S,6R)-cis-α- and (-)-(2S,6R)-cis-γ-irone (Bugoni et al. Chemistry, 2015 Jan 7;21(2):791-9). The difficulties in chemical synthesis highlight the interest to develop procedures of irone production by synthetic biology.

### SUMMARY OF THE INVENTION

The inventors herein demonstrated that irone biosynthetic pathway may be expressed in a recombinant microbial host cell leading to the production of α-irone and/or β-irone.

Accordingly, in a first aspect, the present invention relates to a method of producing an irone compound comprising culturing a recombinant microbial host cell comprising a heterologous nucleic acid encoding a lycopene cyclase catalyzing the β- or ε-cyclization of one or both ends of 2-methyl-lycopene and/or the β- or ε-cyclization of one or both ends of 2,2'-dimethyl-lycopene, under conditions suitable to produce said irone compound and optionally recovering said irone compound.

The lycopene cyclase may be selected from the group consisting of wild-type cyanobacterial CrtL-type lycopene ε-cyclases, wild-type bacterial CrtY-type lycopene β-cyclases and wild-type heterodimeric-type lycopene cyclases of Gram positive bacteria, and variants thereof exhibiting lycopene cyclase activity and having at least 70% sequence identity to any of these cyclases. Preferably, the lycopene cyclase may comprise, or consist of, an amino acid sequence selected from the group consisting of SEQ ID NO: 10, 8, 9, 11, 1, 2 and 3 and variants thereof exhibiting lycopene cyclase activity and having at least 70 %, preferably at least 80%, more preferably at least 90%, 95%, 96%, 97%, 98% or 99%, sequence identity to any of SEQ ID NO: 10, 8, 9, 11, 1, 2 and 3.

In particular, the lycopene cyclase may comprise, or consist of, an amino acid sequence selected from the group consisting of SEQ ID NO: 8 to 11, and variants thereof exhibiting lycopene cyclase activity and having at least 70 %, preferably at least 80%, more preferably at least 90%, 95%, 96%, 97%, 98% or 99%, sequence identity to any of SEQ ID NO: 8 to 11. Preferably, the lycopene cyclase comprises, or consists of, an amino acid sequence selected from the group consisting of SEQ ID NO: 10, and variants thereof exhibiting lycopene cyclase activity and having at least 70 %, preferably at least 80%, more preferably at least 90%, 95%, 96%, 97%, 98% or 99%, sequence identity to SEQ ID NO: 10.

Alternatively, the lycopene cyclase may comprise, or consist of, an amino acid sequence selected from the group consisting of SEQ ID NO: 1 to 3, and variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 3.

Preferably, the recombinant microbial host cell further comprises a heterologous nucleic acid encoding a geranyl pyrophosphate (GPP) C6-methyltransferase. In particular, the GPP C6-methyltransferase may comprise, or consist of, an amino acid sequence selected from the group consisting of SEQ ID NO: 17 to 31 and variants thereof exhibiting GPP C6-methyltransferase activity and having at least 70% sequence identity to SEQ ID NO: 17 to 31.

The recombinant microbial host cell may further comprise a heterologous nucleic acid encoding a farnesyl diphosphate (FPP) synthase and/or a heterologous nucleic acid encoding a geranylgeranyl diphosphate (GGPP) synthase and/or a heterologous nucleic acid encoding a polypeptide exhibiting FPP synthase and GGPP synthase activities; and/or a heterologous nucleic acid encoding a phytoene synthase; and/or a heterologous nucleic acid encoding a lycopene-forming phytoene desaturase; and/or a heterologous nucleic acid encoding a carotenoid cleavage dioxygenase.

The recombinant microbial host may have been genetically modified to reduce the activity of an endogenous PgpB phosphatase, an endogenous CpdB phosphatase and/or an endogenous MetJ repressor by comparison to the non-modified microbial host cell.

Preferably, the recombinant microbial host is a bacterium or a yeast.

In a second aspect, the present invention also relates to a recombinant microbial host as defined above.

In another aspect, the present invention further relates to the use of a recombinant microbial host cell of the invention to produce an irone compound.

### BRIEF DESCRIPTION OF THE DRAWINGS

**Figure 1****:** The designed pathway for isomeric irones. For clarity, only carotenoids with *all-E* configuration are shown.
**Figure 2****:** Engineered pathways to produce C₃₁-carotenoids.
**Figure 3****:** Production of methylated C₃₀ carotenoids by expressing *bezA* in *E. coli.* A) Schematic representation of used genetic constructions. B) Extracted ion chromatograms (EICs) of molecular ions of 4,4'-diapolycopene (**1,** M+° *m*/*z* at 400.3121), 4,4'-diaponeurosporene **(2,** M+° *m*/*z* at 402.277), 2 or 2'-methyl-4,4'-diaponeurosporene (**3,** M₊° *m*/*z* at 416.3435) and 2 or 2'-methyl-4,4'-diapo- ζ-carotene (**4,** M₊° *m*/*z* at 418.3591) are shown. C) Structures of the produced C₃₀ and C₃₁ carotenoids.
**Figure 4****:** Production of methylated C₄₀ carotenoids by expressing *bezA* in *E. coli.* A) Schematic representation of used genetic constructions. B) EICs of molecular ions of lycopene (**5,** M+° *mlz* at 536.4377), 2- or 2'-methyl-lycopene (**6,** M+° *mlz* at 550.4533) and 2,2'-dimethyl-lycopene (**7,** M+° *mlz* at 564.4690) are shown.

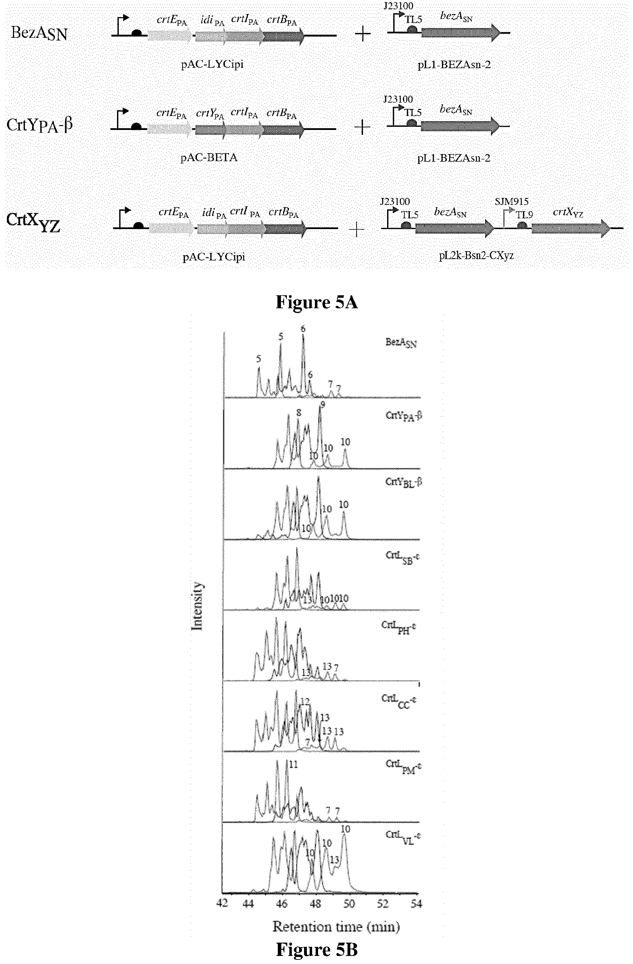

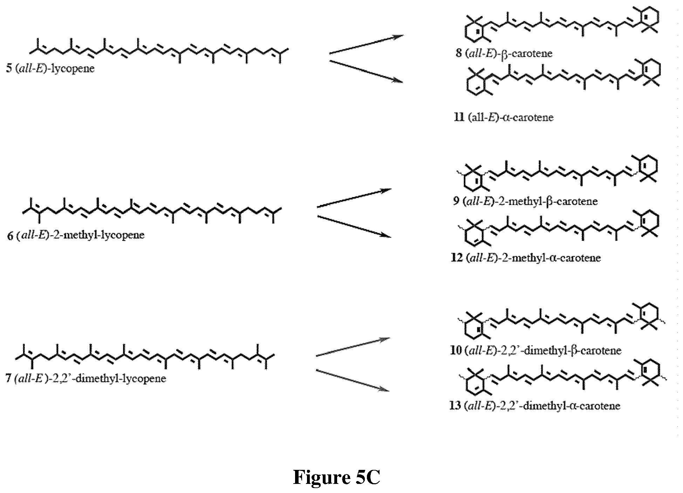

**Figure 5****:** Production of irone-bearing carotene in *E. coli* harboring methyl-lycopene pathway with CrtY-β and CrtL-ε cyclases. A). Schematic representation of used genetic constructions. Respective cyclase gene was cloned with *bezA,* generating a series of plasmids pL2k-Bsn2-CXyz (X and yz depicts the cyclase family and the strain, respectively). For the cyclase CrtYPA-β, the plasmid pAC-BETA was used directly (Cunningham et al. Plant Cell. 8 (1996) 1613-1626). B). EICs of molecular ions M₊° *m*/*z* at 536.4377, 550.4533 and 564.4690 are shown. They correspond to lycopene (5, C40H56) and carotene (**8** or **11,** C40H56), 2-/2'-methyllycopene (**6,** C41H58) and 2-/2'-methyl-carotene (**9** or **12,** C41H58), and 2,2'-dimethyl-lycopene (**7,** C42H56) and 2,2'-dimethyl-carotene (**10** or **13,** C42H56). Annotation of the peaks are deduced from tandem MS analysis. C). Expected carotenoids structures. Only structures with the *E-*configuration are shown.
**Figure 6****:** Production of irones *in vitro* by CCD1_{OF} cleavage. EICs of [M+H]+ ion *m*/*z* at 207.1743, correspond to irone, are shown. The irone standard from Sigma is a racemic mixture of *trans-* and *cis-*α-irone. BezAsn and CXyz depict the recombinant strains from which extracts were used. The corresponding genetic constructions are shown in Figure 5A.
**Figure 7****:** Proposed cycloiridal biosynthetic pathway in the Iris plant leading to various irones by an unknown oxidative process (Belcour et al., Phytochemistry. 34 (1993) 1313-1315 ; Ritzdorf et al. Phytochemistry. 50 (1999) 995-1003).
**Figure 8****:** Chemical structures of (+)-*cis-*α-irone, (-)*-cis*-α-irone*, (+)-trans-α-*irone, *(-)-trans-α-irone,* (+)-β-irone and (-)-β-irone.

### DETAILED DESCRIPTION OF THE INVENTION

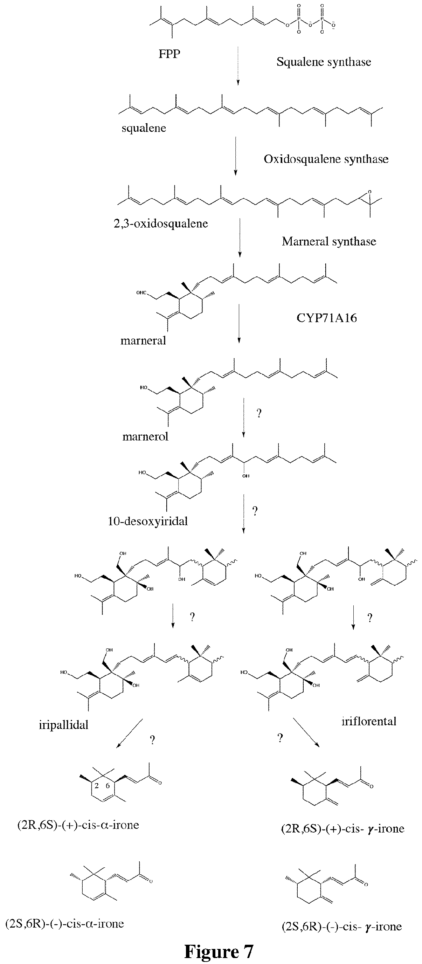

The pathway of irone biogenesis is only partially known in the Iris plants (Figure 7) (Marner et al., Helv. Chim. Acta. 71 (1988) 1331-1338). Irones are in fact not present in fresh rhizomes. They are slowly produced from C₃₁ terpenoid precursors, named cycloiridals, through an elusive oxidative mechanism during the rhizome aging process. Cycloiridal biosynthesis originates from the squalene pathway, although most of the involving enzymes remain unknown (Marner et al., *supra).*

As shown in the experimental section of the present application, the inventors herein demonstrated that irone biosynthetic pathway may be express in a microbial host cell, e.g. *E. coli,* leading to the production of α-irone and β-irone. In particular, they demonstrated that lycopene *β*-cyclases or lycopene ε-cyclases can be used to cyclize mono- and dimethyl-lycopene and to generate irone motif. They also showed that a microbial host cell, e.g. *E. coli,* expressing a 6-GPP methyltransferase can produce a non-canonical C₁₁ building block, 6meGPP, which can be accepted by downstream carotenoid enzymes including CrtE, CrtB and CrtI enzymes to generate mono- and dimethyl-lycopene. After generation of irone motif using lycopene *β-*cyclases or lycopene ε-cyclases, the last step of the designed irone pathway requires cleavage of the double bonds between C9-C10 and C9'-C10' of demethylated carotenes regardless of the configuration of the irone motif. The inventors thus demonstrated the applicability of carotenoid cleavage enzymes (CCDs) in cleavage of nonnatural methylated-carotenes to generate irones thereby producing α-irone and/or β-irone.

### Definitions

In the context of the invention, the term *"recombinant host cell"* designates a cell that is not found in nature and which contains a modified genome as a result of either a deletion, insertion or modification of one or several genetic elements. The term *"host cell"* also encompasses any progeny of a parent host cell that is not identical to the parent host cell due to mutations that occur during replication. Preferably, the host cell is a microbial host cell. As used herein, the term *"microbial host cell"* refers to a bacterium, a filamentous fungus or a yeast, preferably a bacterium or a yeast.

A *"recombinant nucleic acid"* or *"recombinant nucleic acid molecule"* designates a nucleic acid (such as, e.g., DNA, cDNA or RNA molecule) which has been engineered and is not found as such in nature. Typically, this term refers to a nucleic acid molecule comprising segments generated and/or joined together using recombinant DNA technology, such as for example molecular cloning and nucleic acid amplification. A recombinant nucleic acid molecule comprises one or more non-naturally occurring sequences, and/or contains joined nucleic acid molecules from different original sources and not naturally attached together.

The term *"gene"* designates any nucleic acid encoding a protein. This term encompasses DNA, such as cDNA or gDNA, as well as RNA. The gene may be first prepared by e.g., recombinant, enzymatic and/or chemical techniques, and subsequently replicated in a host cell or an *in vitro* system. The gene typically comprises an open reading frame encoding a desired protein. The gene may contain additional sequences such as a transcription terminator or a signal peptide.

The term *"operably linked"* means a configuration in which a control sequence is placed at an appropriate position relative to a coding sequence, in such a way that the control sequence directs expression of the coding sequence.

The term *"control sequences"* means nucleic acid sequences necessary for expression of a gene. Control sequences may be native or heterologous. Well-known control sequences and currently used by the person skilled in the art will be preferred. Such control sequences include, but are not limited to, a leader, polyadenylation sequence, propeptide sequence, promoter, signal peptide sequence, ribosome binding site and transcription terminator. Preferably, the control sequences include a promoter and a transcription terminator.

The term *"expression cassette"* denotes a nucleic acid construct comprising a coding region, i.e. one or several genes, and a regulatory region, i.e. comprising one or more control sequences, operably linked. Optionally, the expression cassette may comprise several coding regions operably linked to several regulatory regions. In particular, the expression cassette may comprise several coding sequences, each of these sequences being operably linked to the same promoter. Alternatively, the expression cassette may comprise one or several coding sequences, each of these sequences operably linked to a distinct promoter. The expression cassette may also comprise one or several coding sequences, each of these sequences operably linked to a distinct promoter and one or several other coding sequences operably linked to a common promoter.

As used herein, the term *"expression vector"* means a DNA or RNA molecule that comprises an expression cassette. Preferably, the expression vector is a linear or circular double stranded DNA molecule. The vector may also comprise an origin of replication, a selection marker, etc.

As used herein, the term *"native"* or *"endogenous",* with respect to a host cell, refers to a genetic element or a protein naturally present in said host cell.

The term *"heterologous",* with respect to a host cell, refers to a genetic element or a protein that is not naturally present in said host cell. The origin of the genetic element or the protein may be different from the cell into which it is introduced. However, the genetic element or the protein may also originate from the same species as the cell into which it is introduced, but it is considered as heterologous on account of its unnatural environment. For example, a genetic element such as a gene, is heterologous since it is under the control of a promoter other than its natural promoter, it is introduced into a position different from that in which it is naturally located. The host cell may contain a copy of the endogenous genetic element or the protein prior to the introduction of the heterologous genetic element or protein or it may not contain an endogenous copy. Moreover, the genetic element or the protein may be heterologous in the sense that the coding sequence has been optimized for expression in the host cell. Codon optimization may be performed via routine processes known in the art (see, for example, Welch, M., et al. (2011), Methods in Enzymology 498: 43-66). Preferably, in the present document, a heterologous nucleic acid sequence codes for a protein which is heterologous to the host cell, i.e. which is not naturally present in said host cell. In particular, this term may refer to a genetic element or a protein provided from a cell of a different species or a different genus than the host cell, more preferably of a different genus than the host cell.

The terms *"peptide", "oligopeptide", "polypeptide"* and *"protein"* are employed interchangeably and refer to a chain of amino acids linked by peptide bonds, regardless of the number of amino acids forming said chain.

The term *"wild-type protein"* as used herein, refers to the non-mutated version of a polypeptide as it appears naturally in a species.

The term *"variant",* as used herein, refers to a polypeptide which is derived from a wild-type protein and comprises an alteration, i.e., a substitution, insertion, and/or deletion, at one or more (e.g., several) positions. The term *"deletion",* used in relation to a position or an amino acid, means that the amino acid in the particular position has been deleted or is absent. The term *"insertion",* used in relation to a position or amino acid, means that one or more amino acids have been inserted or are present adjacent to and immediately following the amino acid occupying the particular position. The term *"substitution",* as used herein, means that an amino acid in a particular position has been replaced by another amino acid or that an amino acid different from the one of the wild-type protein is present. The variant may be obtained by various techniques well known in the art. In particular, examples of techniques for altering the DNA sequence encoding the wild-type protein, include, but are not limited to, site-directed mutagenesis, random mutagenesis and synthetic oligonucleotide construction.

As used herein, the term *"sequence identity"* or *"identity"* refers to the number (%) of matches (identical amino acid residues) in positions from an alignment of two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithms (e.g. Needleman and Wunsch algorithm; Needleman and Wunsch, 1970, J. Mol. Biol 48:443) which aligns the sequences optimally over the entire length, while sequences of substantially different lengths are preferably aligned using a local alignment algorithm (e.g. Smith and Waterman algorithm (Smith & Waterman, Adv. Appl. Math. 2:482, 1981) or Altschul algorithm (Altschul et al. 1997, Nucleic Acids Res. 25:3389-3402; Altschul et al. 2005, FEBS J. 272:5101-5109)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. Preferably, for purposes herein, % amino acid sequence identity values refers to values generated using the BLAST (Basic Local Alignment Search Tool) algorithm, wherein all search parameters are set to default values. In some particular embodiments, all sequence identities (in particular variant sequence identities) are identical and set to at least 70%, 75%, 80%, 85%, 90%, 95%, 96%, 97%, 98% or 99%. In some more particular embodiments, all sequence identities (in particular variant sequence identities) are identical and set to at least 80% sequence identity. In some other particular embodiments, all sequence identities (in particular variant sequence identities) are identical and set to at least 90% or at least 95%, sequence identity. In some embodiments, the variant may contain 1, 2, 3, 4, 5, 6, 7, 8, 9 or 10 additions, substitutions or deletions relative to the wild-type sequence or the sequence described in the SEQ ID NO. In particular, these additions, substitutions or deletions may be introduced at the N-terminal end, the C-terminal end or at both ends. The variant may optionally be in the form of a fusion protein.

The terms *"overexpression"* and *"increased expression"* as used herein are used interchangeably and mean that the expression of a gene or of an enzyme is increased relative to an unmodified microorganism, for example a wild-type microorganism or a microorganism not comprising the genetic modifications described herein. The term *"wild-type"* refers to an unmodified microorganism existing in nature. The increased expression of an enzyme is usually obtained by increasing the expression of the gene coding for said enzyme. In embodiments in which the gene or the enzyme is not naturally present in the host cell of the invention, i.e. a heterologous gene or enzyme, the terms *"overexpression"* and *"expression"* may be used interchangeably. To increase the expression of a gene, a person skilled in the art can use any known technique such as increasing the number of copies of the gene in the microorganism, by using a promoter inducing a high level of expression of the gene, i.e. a strong promoter, by using elements which stabilize the corresponding messenger RNA or sequences which sequester the ribosomal binding site (RBS) and the sequences surrounding same. In particular, overexpression may be obtained by increasing the number of copies of the gene in the microorganism. One or more copies of the gene may be introduced into the genome via recombination processes, known to those skilled in the art, including the replacement of the genes or multi-copy integration. Preferably, an expression cassette comprising the gene, preferably placed under the control of a strong promoter, is integrated into the genome. As an alternative, the gene may be carried by an expression vector, preferably a plasmid, comprising an expression cassette with the gene of interest preferably placed under the control of a strong promoter. The expression vector may be present in the microorganism in one or more copies, depending on the nature of the origin of replication. Overexpression of the gene may also be obtained by using a promoter which induces a high level of expression of the gene. For example, the promoter of an endogenous gene may be replaced with a stronger promoter, i.e. a promoter which induces a higher level of expression. The endogenous gene under the control of a promoter which is not the natural promoter is termed a heterologous nucleic acid. The promoters that are suitable for use in the present invention are known to those skilled in the art and may be constitutive or inducible, and may be endogenous or heterologous.

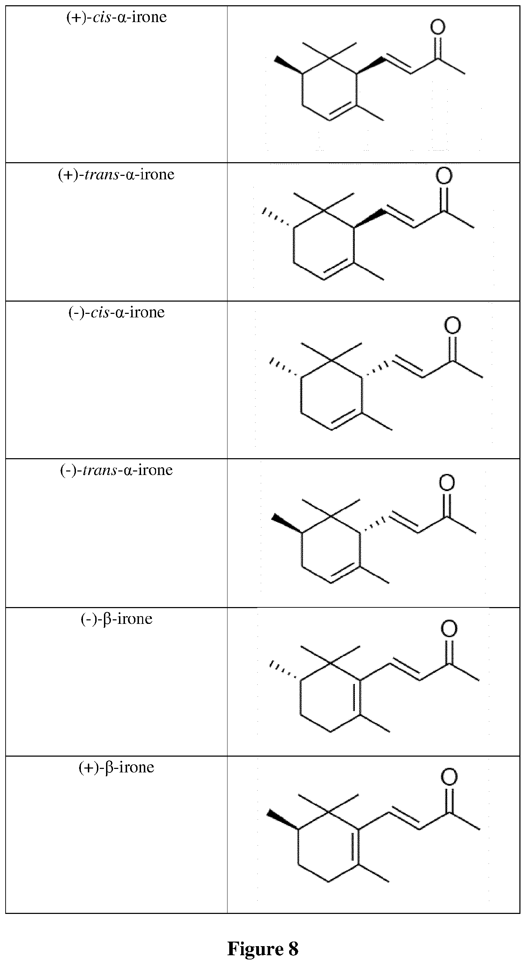

As used herein, the term "irone" or "irone compound" refers to a compound selected from the group consisting of α-irone, β-irone and γ-irone. Preferably, this term refers to a compound selected from the group consisting of α-irone and β-irone. As used herein, the term *"α-irone"* refers to a compound selected from the group consisting of (+)-cis-α-irone, (-)-cis-α-irone, (+)-trans-α-irone, (-)-trans-α-irone, and any mixture thereof. As used herein, the term *"β-irone"* refers to a compound selected from the group consisting of (+)-β-irone and (-)-β-irone, and a mixture thereof. These compounds are represented in Figure 8.

### Recombinant microbial host cell

The inventors herein demonstrated that lycopene *β*-cyclases or lycopene ε-cyclases can be used to cyclize mono- and dimethyl-lycopene and to generate irone motif in a recombinant microbial host cell, in particular in *E. coli,* thereby paving the way for the production of bio-based irone compounds.

Thus, in a first aspect, the present invention relates to a recombinant microbial host cell comprising a heterologous nucleic acid encoding a lycopene cyclase catalyzing the production of 2-methyl-carotene from 2-methyl-lycopene and/or the production of 2,2'-dimethyl-carotene from 2,2'-dimethyl-lycopene.

The microbial host cell of the present invention may be a eukaryotic microorganism selected from the group consisting of yeasts and filamentous fungi, or may be a prokaryotic microorganism.

In an embodiment, the microbial host cell is a yeast, preferably selected from the yeasts of the Saccharomycetales, Sporidiobolales and Schizosaccharomycetales orders. In particular, the yeast may be selected, for example, from the group consisting of *Pichia, Kluyveromyces, Saccharomyces, Schizosaccharomyces, Candida, Lipomyces, Rhodotorula, Rhodosporidium, Yarrowia,* or *Debaryomyces* yeasts. Preferably, the yeast is selected from the group consisting of *Pichia pastoris, Kluyveromyces lactis, Kluyveromyces marxianus, Saccharomyces cerevisiae, Saccharomyces carlsbergensis, Saccharomyces diastaticus, Saccharomyces douglasii, Saccharomyces kluyveri, Saccharomyces norbensis, Saccharomyces oviformis, Schizosaccharomyces pombe, Candida albicans, Candida tropicalis, Rhodotorula glutinis, Rhodosporidium toruloides, Yarrowia lipolytica, Debaryomyces hansenii* and *Lipomyces starkeyi.* More preferably, the yeast is a *Saccharomyces* yeast, preferably a *Saccharomyces cerevisiae* yeast.

In another embodiment, the microbial host cell is a filamentous fungus, i.e. a fungus growing in the form of mycelium of hyphae. The filamentous fungi are generally characterized by a mycelial wall composed of chitin, cellulose, glucan, chitosan, mannan, and other complex polysaccharides. The filamentous fungus is preferably selected from the group consisting of fungi of the genera *Acremonium, Aspergillus, Aureobasidium, Bjerkandera, Ceriporiopsis, Chrysosporium, Cochiobolus, Coprinus, Coriolus, Cryptococcus, Endothia, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Podospora, Pyricularia, Schizophyllum, Talaromyces, Thermoascus, Thielavia, Tolypocladium, Trametes* and *Trichoderma.* Preferably, the filamentous fungus is selected from fungi of the genus *Aspergillus, Neurospora* or *Trichoderma.* In particular, the filamentous fungus may be selected from the group consisting of *Aspergillus nidulans, Aspergillus niger, Aspergillus awomari, Aspergillus oryzae, Aspergillus terreus, Neurospora crassa, Trichoderma reesei* and *Trichoderma viride.*

In another embodiment, the microbial host cell is a prokaryote, preferably a bacterium. In particular, the bacterium may be selected from the group consisting of the bacteria from the phylum *Acidobacteria, Actinobacteria, Aquificae, Bacterioidetes, Chlamydiae, Chlorobi, Chloroflexi, Chrysiogenetes, Cyanobacteria, Deferribacteres, Deinococcus-Thermus, Dictyoglomi, Fibrobacteres, Firmicutes, Fusobacteria, Gemmatimonadetes, Nitrospirae, Planctomycetes, Proteobacteria, Spirochaetes, Thermodesulfobacteria, Thermomicrobia, Thermotogae* or *Verrucomicrobia.* Preferably, the bacterium belongs to the genus *Acaryochloris, Acetobacter, Actinobacillus, Agrobacterium, Alicyclobacillus, Anabaena, Anacystis, Anaerobiospirillum, Aquifex, Arthrobacter, Arthrospira, Azobacter, Bacillus, Brevibacterium, Burkholderia, Chlorobium, Chromatium, Chlorobaculum, Clostridium, Corynebacterium, Cupriavidus, Cyanothece, Enterobacter, Deinococcus, Erwinia, Escherichia, Geobacter, Gloeobacter, Gluconobacter, Hydrogenobacter, Klebsiella, Lactobacillus, Lactococcus, Mannheimia, Mesorhizobium, Methylobacterium, Microbacterium, Microcystis, Nitrobacter, Nitrosomonas, Nitrospina, Nitrospira, Nostoc, Phormidium, Prochlorococcus, Pseudomonas, Ralstonia, Rhizobium, Rhodobacter, Rhodococcus, Rhodopseudomonas, Rhodospirillum, Salmonella, Scenedesmun, Serratia, Shigella, Staphylococcus, Streptomyces, Synechoccus, Synechocystis, Thermosynechococcus, Trichodesmium* or *Zymomonas.* More preferably, the bacterium is selected from the group consisting of *Agrobacterium tumefaciens, Anaerobiospirillum succiniciproducens, Actinobacillus succinogenes, Aquifex aeolicus, Aquifex pyrophilus, Bacillus subtilis, Bacillus amyloliquefacines, Brevibacterium ammoniagenes, Brevibacterium immariophilum, Clostridium pasteurianum, Clostridium ljungdahlii, Clostridium acetobutylicum, Clostridium beigerinckii, Corynebacterium glutamicum, Cupriavidus necator, Cupriavidus metallidurans, Enterobacter sakazakii, Escherichia coli, Gluconobacter oxydans, Hydrogenobacter thermophilus, Klebsiella oxytoca, Lactococcus lactis, Lactobacillus plantarum, Mannheimia succiniciproducens, Mesorhizobium loti, Pseudomonas aeruginosa, Pseudomonas mevalonii, Pseudomonas pudica, Pseudomonas putida, Pseudomonas fluorescens, Rhizobium etli, Rhodobacter capsulatus, Rhodobacter sphaeroides, Rhodospirillum rubrum, Salmonella enterica, Salmonella typhi, Salmonella typhimurium, Shigella dysenteriae, Shigella flexneri, Shigella sonnei, Staphylococcus aureus, Streptomyces coelicolor, Zymomonas mobilis, Acaryochloris marina, Anabaena variabilis, Arthrospira platensis, Arthrospira maxima, Chlorobium tepidum, Chlorobaculum sp., Cyanothece sp., Gloeobacter violaceus, Microcystis aeruginosa, Nostoc punctiforme, Prochlorococcus marinus, Synechococcus elongatus, Synechocystis sp., Thermosynechococcus elongatus, Trichodesmium erythraeum* and *Rhodopseudomonas palustris.* In a particular embodiment, the microorganism is a bacterium which does not belong to the *Streptomyces* genus. In a preferred embodiment, the microorganism is an *Escherichia coli* bacterium.

In a particular embodiment, the microbial host cell is selected from the group consisting of yeasts, filamentous fungi and bacteria which do not belong to the *Streptomyces* genus. Preferably, the microbial host cell is a yeast, a filamentous fungi or an *Escherichia coli* bacterium.

### Lycopene cyclase activity

The recombinant microbial host cell of the invention comprises a heterologous nucleic acid encoding a lycopene cyclase catalyzing the P- or ε-cyclization of one or both ends of 2-methyl-lycopene and/or the P- or ε-cyclization of one or both ends of 2,2'-dimethyl-lycopene.

As used herein, the term *"lycopene cyclase"* refers to an enzyme exhibiting lycopene β-cyclase activity (EC 5.5.1.19) and/or lycopene ε-cyclase activity (EC 5.5.1.18), i.e. an enzyme that catalyzes the β-cyclization of one end of lycopene to form γ-carotene, the β-cyclization of both ends of lycopene to produce β-carotene, the ε-cyclization of one end of lycopene to form δ-carotene and/or the ε-cyclization of both ends of lycopene to produce ε-carotene. In the context of the present invention, the term *"lycopene cyclase activity"* refers to β- or ε-cyclization of one or both ends of 2-methyl-lycopene, preferably of both ends of 2-methyl-lycopene to yield 2-methyl-carotene, or the β- or ε-cyclization of one or both ends of 2,2'-dimethyl-lycopene, preferably of both ends of 2,2'-dimethyl-lycopene to yield 2,2'-dimethyl-carotene. Lycopene cyclase activity may be assessed by any method known by the skilled person. For example, this activity may be assessed by co-expressing lycopene cyclase gene together with *crtE, crtB,* and *crtI* genes in a host cell, e.g. *E. coli,* coupled to subsequent pigment analysis. The pigment is extracted and analysed by LC coupled to UV/Vis spectroscopic detection and/or with MS analysis. The detection of products of the β- or ε-cyclization of 2-methyl-lycopene, preferably 2-methyl-carotene, and/or of the β- or ε-cyclization of 2,2'-dimethyl-lycopene, preferably 2,2'-dimethyl-carotene indicates that the tested enzyme exhibits lycopene cyclase activity.

Lycopene cyclases may be divided into four classes: the bacterial CrtY-type lycopene β-cyclases that are found in particular in many carotenogenic proteobacteria (e.g., Misawa et al. J Bacteriol, 172, 6704-6712, 1990; Matsumura et al. Gene 189, 169-174, 1997), *Streptomyces* spp. (Krugel et al. Biochim Biophys Acta 1439, 57-64, 1999), and the Chloroflexi; the CrtL-type lycopene cyclases which include the β- and ε-cyclases in some cyanobacteria, algae and plants (Cunningham et al. Plant Cell 6, 1107-1121, 1994; Stickforth et al. Arch Microbiol 179, 409-415, 2003); a class comprising the heterodimeric cyclases (CrtYc/CrtYd) of some Gram-positive bacteria (Krubasik et al. Mol Gen Genet 263, 423-432, 2000; Viveiros et al. FEMS Microbiol Lett 187, 95-101, 2000), the monomeric cyclase CrtYc-Yd in archaea and the fused and bifunctional cyclases CrtYB in fungi (Hemmi et al. , 2003. Biochem. Biophys. Res. Commun., 35, 586-591; Takaichi et al. 2011. Mar. Drugs, 9, 1101-1118); and the lycopene cyclases of the CruA/CruP family which are found in green sulfur bacteria and cyanobacteria that do not possess CrtY or CrtL cyclase (Takaichi et al., 2013, Tetraterpenes: carotenoids. In Natural Products, ed. by Merillon, J. M. and Ramawat, K. G., Springer, Berlin pp. 3251-3283).

Examples of lycopene cyclases include, but are not limited to, lycopene cyclases from *Brevibacterium linens* (CrtY_{BL}-β ; heterodimer CrtYc/CrtYd, Uniprot accession number for CrtYc: Q9KK78, SEQ ID NO: 1, and Uniprot accession number for CrtYd: Q9KK79, SEQ ID NO: 2), from *Pantoe agglomerans* (CrtY_{PA}-β, Uniprot accession number: K7WHX6, SEQ ID NO: 3), from *Porphyra umbilicalis* (GenBank accession no : QHA79699.1, SEQ ID NO: 4), from *Synechococcus elongatus* (Uniprot accession number: Q55276, SEQ ID NO: 5) and from yeast *Phaffia rhodozyma* (Uniprot accession number: Q7Z859, SEQ ID NO: 6), from *Vulcanococcus limneticus* (CrtL_{VL}-ε, NCBI Reference Sequence: WP_094588021, SEQ ID NO: 8), *Synechococcus* sp. BS55DK (CrtL_{SB}-ε, NCBI Reference Sequence: WP_131594774, SEQ ID NO: 9), *Cyanobium* sp. CACIAM 14 (CrtL_{CC}-ε, NCBI GenBank accession number: KEF41217, SEQ ID NO: 10), *Prochlorococcus* sp. HOT208 (CrtL_{PH}-c, NCBI Reference Sequence: WP_079293938, SEQ ID NO: 11), *Porphyra umbilicalis* (GenBank accession no. QHA79700.1, SEQ ID NO: 12), *Oryza sativa* (NCBI Reference Sequence: XP_015622198.1, SEQ ID NO: 13), *Zea mays* (Uniprot accession number: B7S825, SEQ ID NO: 14), *Chromochloris zofingiensis* (Uniprot accession number: J7Q2X9, SEQ ID NO: 15) and *Haematococcus lacustris* (GenBank accession number : AKT95178.1, SEQ ID NO: 16). Other lycopene cyclases can be easily identified using well-known databases or any sequence alignment software applied on the lycopene cyclases listed above.

In particular, the lycopene cyclase may comprise, or consist of, an amino acid sequence selected from the group consisting of SEQ ID NO: 1 to 6 and 8 to 16 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 6 and 8 to 16. In particular, said variants may comprise, or consist of, a sequence that differs from a sequence set forth in any of SEQ ID No. 1 to 6 and 8 to 16 by 1, 2, 3, 4, 5, 6, 7, 8, 9 or 10 substitutions, insertions and/or deletions, preferably by 1, 2, 3, 4 or 5 substitutions, insertions and/or deletions.

Preferably, the lycopene cyclase may comprise, or consist of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11. In particular, said variants may comprise, or consist of, a sequence that differs from a sequence set forth in any of SEQ ID No. 1 to 3 and 8 to 11 by 1, 2, 3, 4, 5, 6, 7, 8, 9 or 10 substitutions, insertions and/or deletions, preferably by 1, 2, 3, 4 or 5 substitutions, insertions and/or deletions.

In an embodiment, the recombinant microbial host cell of the invention comprises a heterologous nucleic acid encoding a lycopene β-cyclase.

As used herein, the term *"lycopene β-cyclase"* refers to an enzyme that catalyzes the β-cyclization of one end of lycopene to form γ-carotene or the β-cyclization of both ends of lycopene to produce β-carotene (EC 5.5.1.19). In the context of the present invention, the term *"lycopene β-cyclase activity"* refers to the β-cyclization of one or both ends of 2-methyl-lycopene, preferably of both ends of 2-methyl-lycopene to yield 2-methyl-β-carotene, or the β-cyclization of one or both ends of 2,2'-dimethyl-lycopene, preferably of both ends of 2,2'-dimethyl-lycopene to yield 2,2'-dimethyl-β-carotene. Lycopene β-cyclase activity may be assessed by any method known by the skilled person. For example, this activity may be assessed by co-expressing lycopene cyclase gene together with *crtE, crtB,* and *crtI* genes in a host cell, e.g. *E. coli,* coupled to subsequent pigment analysis. The pigment is extracted and analysed by LC coupled to UV/Vis spectroscopic detection and/or with MS analysis. The detection of products of the β-cyclization of 2-methyl-lycopene, preferably 2-methyl-β-carotene, and/or of the β-cyclization of 2,2'-dimethyl-lycopene, preferably 2,2'-dimethyl-β-carotene indicates that the tested enzyme exhibits lycopene β-cyclase activity.

Examples of lycopene β-cyclases include, but are not limited to, lycopene β-cyclases from *Brevibacterium linens* (CrtY_{BL}-β ; heterodimer CrtYc/CrtYd, SEQ ID NO: 1 and CrtYd SEQ ID NO: 2), from *Pantoe agglomerans* (CrtY_{PA}-β, SEQ ID NO: 3), from *Porphyra umbilicalis* (SEQ ID NO: 4), from *Synechococcus elongatus* (SEQ ID NO: 5) and from yeast *Phaffia rhodozyma* (SEQ ID NO: 6). Other lycopene β-cyclases can be easily identified using well-known databases or any sequence alignment software applied on the lycopene β-cyclases listed above.

In particular, the lycopene β-cyclase may comprise, or consist of, an amino acid sequence selected from the group consisting of SEQ ID NO: 1 to 6 and variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 6. In particular, said variants may comprise, or consist of, a sequence that differs from a sequence set forth in any of SEQ ID No. 1 to 6 by 1, 2, 3, 4, 5, 6, 7, 8, 9 or 10 substitutions, insertions and/or deletions, preferably by 1, 2, 3, 4 or 5 substitutions, insertions and/or deletions.

In a preferred embodiment, the lycopene cyclase is a lycopene β-cyclase selected from the group consisting of wild-type bacterial CrtY-type lycopene β-cyclases and wild-type heterodimeric-type lycopene β-cyclases of Gram positive bacteria, and the variants thereof exhibiting lycopene β-cyclase activity and having at least 70 %, preferably at least 80%, more preferably at least 90%, 95%, 96%, 97%, 98% or 99%, sequence identity to any of these cyclases.

Examples of bacterial CrtY-type lycopene β-cyclases include, but are not limited to, *Pantoe agglomerans* (CrtY_{PA}-β, SEQ ID NO: 3).

Examples of heterodimeric-type lycopene cyclases of Gram positive bacteria include, but are not limited to, lycopene cyclases from *Brevibacterium linens* (SEQ ID NO: 1 and 2).

In a particular embodiment, the lycopene cyclase comprises, or consists of, an amino acid sequence selected from the group consisting of SEQ ID NO: 1 to 3, and the variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 3. In particular, said variants may comprise, or consist of, a sequence that differs from a sequence set forth in any of SEQ ID No. 1 to 3 by 1, 2, 3, 4, 5, 6, 7, 8, 9 or 10 substitutions, insertions and/or deletions, preferably by 1, 2, 3, 4 or 5 substitutions, insertions and/or deletions.

In a more particular embodiment, the lycopene cyclase comprises, or consists of, (i) an amino acid sequence selected from the group consisting of SEQ ID NO: 3 and the variants thereof exhibiting lycopene β-cyclase activity and having at least 70 %, preferably at least 80%, more preferably at least 90%, 95%, 96%, 97%, 98% or 99%, sequence identity to SEQ ID NO:3 and (ii) a heterodimeric lycopene cyclase comprising a first subunit comprising, or consisting of, an amino acid sequence selected from the group consisting of SEQ ID NO: 1 and the variants thereof exhibiting lycopene β-cyclase activity and having at least 70 %, preferably at least 80%, more preferably at least 90%, 95%, 96%, 97%, 98% or 99%, sequence identity to SEQ ID NO:1, and a second subunit comprising, or consisting of, an amino acid sequence selected from the group consisting of SEQ ID NO: 2 and the variants thereof exhibiting lycopene β-cyclase activity and having at least 70 %, preferably at least 80%, more preferably at least 90%, 95%, 96%, 97%, 98% or 99%, sequence identity to SEQ ID NO:2.

In another embodiment, the recombinant microbial host cell of the invention comprises a heterologous nucleic acid encoding a lycopene ε-cyclase.

As used herein, the term *"lycopene* ε*-cyclase"* refers to an enzyme that catalyzes the ε-cyclization of one end of lycopene to form δ-carotene or the ε-cyclization of both ends of lycopene to produce ε-carotene (EC 5.5.1.18). In the context of the present invention, the term *"lycopene* ε*-cyclase activity"* refers the ε-cyclization of one or both ends of 2-methyl-lycopene, preferably of both ends of 2-methyl-lycopene to yield 2-methyl-α-carotene, or the ε-cyclization of one or both ends of 2,2'-dimethyl-lycopene, preferably of both ends of 2,2'-dimethyl-lycopene to yield 2,2'-dimethyl-α-carotene. Lycopene ε-cyclase activity may be assessed by any method known by the skilled person. For example, this activity may be assessed by co-expressing lycopene cyclase gene together with *crtE, crtB,* and *crtI* genes in a host cell, e.g. *E. coli,* coupled to subsequent pigment analysis. The pigment is extracted and analysed by LC coupled to UV/Vis spectroscopic detection and/or with MS analysis. The detection of products of the ε-cyclization of 2-methyl-lycopene, preferably 2-methyl-α-carotene, and/or of the ε-cyclization of 2,2'-dimethyl-lycopene, preferably 2,2'-dimethyl-α-carotene indicates that the tested enzyme exhibits lycopene ε-cyclase activity. Some lycopene ε-cyclases may further exhibit lycopene β-cyclase activity as defined above, in particular may produce 2-methyl-β-carotene and/or 2,2'-dimethyl-β-carotene, preferably 2,2'-dimethyl-β-carotene.

Examples of lycopene ε-cyclases include, but are not limited to, lycopene ε-cyclases from *Vulcanococcus limneticus* (CrtL_{VL}-ε, SEQ ID NO: 8), *Synechococcus* sp. BS55DK (CrtL_{SB}-ε, SEQ ID NO: 9), *Cyanobium* sp. CACIAM 14 (CrtL_{CC}-ε, NCBI SEQ ID NO: 10), *Prochlorococcus* sp. HOT208 (CrtL_{PH}-ε, SEQ ID NO: 11), *Porphyra umbilicalis* (SEQ ID NO: 12), *Oryza sativa* (SEQ ID NO: 13), *Zea mays* (SEQ ID NO: 14), *Chromochloris zofingiensis* (SEQ ID NO: 15) and *Haematococcus lacustris* (SEQ ID NO: 16). Other lycopene ε-cyclases can be easily identified using well-known databases or any sequence alignment software applied on the lycopene ε-cyclases listed above.

In particular, the lycopene ε-cyclase may comprise, or consist of, an amino acid sequence selected from the group consisting of SEQ ID NO: 8 to 16 and variants thereof exhibiting lycopene cyclase activity, preferably lycopene ε-cyclase activity and optionally lycopene β-cyclase activity, and having at least 70 %, preferably at least 80%, more preferably at least 90%, 95%, 96%, 97%, 98% or 99%, sequence identity to any of SEQ ID NO: 8 to 16. In particular, said variants may comprise, or consist of, a sequence that differs from a sequence set forth in any of SEQ ID No. 8 to 16 by 1, 2, 3, 4, 5, 6, 7, 8, 9 or 10 substitutions, insertions and/or deletions, preferably by 1, 2, 3, 4 or 5 substitutions, insertions and/or deletions.

In a preferred embodiment, the lycopene cyclase is a lycopene ε-cyclase selected from the group consisting of wild-type cyanobacterial CrtL-type ε-cyclases , and the variants thereof exhibiting lycopene cyclase activity, preferably lycopene ε-cyclase activity and optionally lycopene β-cyclase activity, and having at least 70 %, preferably at least 80%, more preferably at least 90%, 95%, 96%, 97%, 98% or 99%, sequence identity to any of these cyclases.

Examples of cyanobacterial CrtL-type lycopene ε-cyclases include, but are not limited to, lycopene ε-cyclases from *Vulcanococcus limneticus* (CrtL_{VL}-ε, SEQ ID NO: *8), Synechococcus* sp. BS55DK (CrtL_{SB}-ε, SEQ ID NO: 9), *Cyanobium* sp. CACIAM 14 (CrtL_{CC}-ε, NCBI SEQ ID NO: 10) and *Prochlorococcus* sp. HOT208 (CrtL_{PH}-ε, SEQ ID NO: 11).

In a particular embodiment, the lycopene cyclase comprises, or consists of, an amino acid sequence selected from the group consisting of SEQ ID NO: 8 to 11, and the variants thereof exhibiting lycopene cyclase activity, preferably lycopene ε-cyclase activity and optionally lycopene β-cyclase activity, and having at least 70 %, preferably at least 80%, more preferably at least 90%, 95%, 96%, 97%, 98% or 99%, sequence identity to any of SEQ ID NO: 8 to 11. In particular, said variants may comprise, or consist of, a sequence that differs from a sequence set forth in any of SEQ ID No. 8 to 11 by 1, 2, 3, 4, 5, 6, 7, 8, 9 or 10 substitutions, insertions and/or deletions, preferably by 1, 2, 3, 4 or 5 substitutions, insertions and/or deletions.

In a particular embodiment, the irone compound is β-irone and the lycopene cyclase is selected from the group consisting of lycopene β-cyclases and lycopene ε-cyclases exhibiting lycopene β-cyclase activity, preferably selected from the group consisting of wild-type bacterial CrtY-type lycopene β-cyclases, wild-type heterodimeric-type lycopene β-cyclases of Gram positive bacteria, and wild-type cyanobacterial CrtL-type lycopene ε-cyclases exhibiting lycopene β-cyclase activity, and the variants thereof exhibiting lycopene β-cyclase activity and having at least 70 %, preferably at least 80%, more preferably at least 90%, 95%, 96%, 97%, 98% or 99%, sequence identity to any of these cyclases. Preferably, the lycopene cyclase comprises, or consists of, an amino acid sequence selected from the group consisting of SEQ ID NO: 1 to 6, 8 and 9, and the variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 6, 8 and 9. More preferably, the lycopene cyclase comprises, or consists of, an amino acid sequence selected from the group consisting of SEQ ID NO: 1 to 3, 8 and 9, and the variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 3, 8 and 9. In particular, said variants may comprise, or consist of, a sequence that differs from a sequence set forth in any of SEQ ID NO: 1 to 6, 8 and 9, preferably from a sequence set forth in any of SEQ ID NO: 1 to 3, 8 and 9, by 1, 2, 3, 4, 5, 6, 7, 8, 9 or 10 substitutions, insertions and/or deletions, preferably by 1, 2, 3, 4 or 5 substitutions, insertions and/or deletions.

In another particular embodiment, the irone compound is α-irone and the lycopene cyclase is selected from the group consisting of lycopene ε-cyclases, preferably from the group consisting of wild-type cyanobacterial CrtL-type lycopene ε-cyclases, and the variants thereof exhibiting lycopene ε-cyclase activity and having at least 70 %, preferably at least 80%, more preferably at least 90%, 95%, 96%, 97%, 98% or 99%, sequence identity to any of these cyclases. Preferably, the lycopene cyclase comprises, or consists of, an amino acid sequence selected from the group consisting of SEQ ID NO: 8 to 16, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 8 to 16. More preferably, the lycopene cyclase comprises, or consists of, an amino acid sequence selected from the group consisting of SEQ ID NO: 8 to 11, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 8 to 11. Even more preferably, the lycopene cyclase comprises, or consists of, an amino acid sequence selected from the group consisting of SEQ ID NO: 10 and 11, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 10 and 11. In particular, said variants may comprise, or consist of, a sequence that differs from a sequence set forth in any of SEQ ID NO: 8 to 16, preferably from a sequence set forth in any of SEQ ID NO: 8 to 11, more preferably from a sequence set forth in any of SEQ ID NO: 10 and 11, by 1, 2, 3, 4, 5, 6, 7, 8, 9 or 10 substitutions, insertions and/or deletions, preferably by 1, 2, 3, 4 or 5 substitutions, insertions and/or deletions.

In another particular embodiment, the irone compound is cis-α-irone and the lycopene cyclase comprises, or consists of, an amino acid sequence selected from the group consisting of SEQ ID NO: 10, and the variants thereof exhibiting lycopene ε-cyclase activity and having at least 70 %, preferably at least 80%, more preferably at least 90%, 95%, 96%, 97%, 98% or 99%, sequence identity to SEQ ID NO: 10. In particular, said variants may comprise, or consist of, a sequence that differs from a sequence set forth in SEQ ID NO: 10, by 1, 2, 3, 4, 5, 6, 7, 8, 9 or 10 substitutions, insertions and/or deletions, preferably by 1, 2, 3, 4 or 5 substitutions, insertions and/or deletions.

The recombinant microbial host cell of the invention may naturally express said lycopene cyclase. In this case, said recombinant host cell may be genetically modified to overexpress this protein or to further express a heterologous lycopene cyclase. In embodiments wherein the recombinant microbial host cell does not naturally express said lycopene cyclase, said host cell is genetically modified to express said heterologous lycopene cyclase. In preferred embodiments, the host cell is genetically modified to express a heterologous lycopene cyclase.

### Production of non-canonical building blocks

The recombinant microbial host cell of the invention is able to produce the non-canonical building block 14-methylgeranylgeranyl diphosphate (14-meGGPP) which can be accepted by downstream carotenoid enzymes including CrtB and CrtI enzymes to generate mono- and dimethyl-lycopene.

In preferred embodiments, the recombinant microbial host cell of the invention exhibits geranyl pyrophosphate (GPP) C6-methyltransferase activity. Said geranyl pyrophosphate (GPP) C6-methyltransferase catalyzes a methylation of GPP to yield 6-methylgeranyl diphosphate (6-meGPP); 6-meGPP is then converted by farnesyl diphosphate synthase and CrtE enzyme to generate 14-meGGPP.

As used herein, the term *"geranyl pyrophosphate C6-methyltransferase", "geranyl pyrophosphate (GPP) C6-methyltransferase"* or *"GPP C6-methyltransferase"* refers to an enzyme that catalyzes a S-adenosylmethionine (SAM)-dependent methylation of geranyl diphosphate (GPP) to yield 6-methylgeranyl diphosphate (6-meGPP). GPP C6-methyltransferase activity may be assessed by any method known by the skilled person. For example, this activity may be assessed by incubating a putative GPP C6-methyltransferase with GPP, SAM and Mg²⁺ and detecting the production of 6-meGPP using any suitable method such as liquid chromatography coupled to electrospray ionization mass spectrometry (LC-ESI-MS) analysis in negative mode (see e.g. Lee et al. 2011. Mass Spectrometry Letters, 2(4), 92-95). The position of the methyl group can be confirmed by coupled use of me-GPP derivatization (pyrophosphate hydrolysis followed by epoxidation of double bonds) and LC-tandem MS analysis (see e.g. Tsutsumi et al. Angew Chem Int Ed Engl. 2022 Jan 3;61(1):e202111217).

The GPP C6-methyltransferase may be selected from known GPP C6-methyltransferases and variants thereof exhibiting GPP C6-methyltransferase activity and having at least 70 %, preferably at least 80%, more preferably at least 90%, 95%, 96%, 97%, 98% or 99%, sequence identity to any of said known GPP C6-methyltransferases.

Examples of GPP C6-methyltransferases include, but are not limited to, BezA enzyme from the benzastatin biosynthetic pathway of *Streptomyces niveus* (SEQ ID NO: 17), BezA of *Streptomyces* sp. RI18 (SEQ ID NO: 18, Tsutsumi et al., Angew Chem Int Ed Engl. 2022 Jan 3;61(1):e202111217, J. Am. Chem. Soc. 2018, 140, 21, 6631-6639), homologues of BezA from *Streptomyces* sp. 4R-3d (SEQ ID NO: 19), *Streptomyces ipomoeae* (SEQ ID NO: 20), *Streptomyces malaysiensis* (SEQ ID NO: 21), *Streptomyces* sp. (SEQ ID NO: 22), *Streptomyces antimycoticus* (SEQ ID NO: 23), *Streptomyces antioxidans* (SEQ ID NO: 24), *Streptomyces* sp. 150FB (SEQ ID NO: 25), *Streptomyces caeruleatus* (SEQ ID NO: 26), *Streptomyces adelaidensis* (SEQ ID NO: 27), *Streptomyces kasugaensis* (SEQ ID NO: 28), *Streptomyces acidiscabies* (SEQ ID NO: *29), Lentzea indica* (SEQ ID NO: 30) and *Saccharothrix espanaensis* (SEQ ID NO: 31).

Preferably, the GPP C6-methyltransferase comprises, or consists of, an amino acid sequence selected from the group consisting of SEQ ID NO: 17 to 31, and variants thereof exhibiting GPP C6-methyltransferase activity and having at least 70 %, preferably at least 80%, more preferably at least 90%, 95%, 96%, 97%, 98% or 99%, sequence identity to any of SEQ ID NO: 17 to 31. In particular, said variants may comprise, or consist of, a sequence that differs from a sequence set forth in any of SEQ ID No. 1 to 15 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the GPP C6-methyltransferase comprises, or consists of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17. In particular, said variants may comprise, or consist of, a sequence that differs from the sequence of SEQ ID NO. 17 or 18, preferably SEQ ID NO: 1, by 1, 2, 3, 4, 5, 6, 7, 8, 9 or 10 substitutions, insertions and/or deletions, preferably by 1, 2, 3, 4 or 5 substitutions, insertions and/or deletions.

In preferred embodiments, substitutions, insertions and/or deletions of the variants of GPP C6-methyltransferase do not affect residues of said enzymes known as important for catalysis, phosphate binding and substrate specificity, in particular residues of said enzymes corresponding to Y28, K31, K38, Y47, E170, Y174, W210, Y213 F214, S277 or N281 of SEQ ID NO: 18. The residue corresponding to Y28, K31, K38, Y47, E170, Y174, W210, Y213 F214, S277 or N281 of SEQ ID NO: 18 in a sequence of a GPP C6-methyltransferase can be readily identified by conventional sequence alignment techniques.

The recombinant microbial host cell of the invention may naturally express a GPP C6-methyltransferase, e.g. when the host cell is a *Streptomyces* bacterium. In this case, said recombinant host cell may be genetically modified to overexpress the endogenous GPP C6-methyltransferase or to further express a heterologous GPP C6-methyltransferase. In preferred embodiments, in particular when the recombinant microbial host cell does not naturally express a GPP C6-methyltransferase, said host cell is genetically modified to express a heterologous GPP C6-methyltransferase.

In a particular embodiment, the recombinant microbial host cell comprises
- a heterologous nucleic acid encoding a lycopene cyclase, preferably a lycopene cyclase comprising, or consisting of, an amino acid sequence selected from the group consisting of SEQ ID NO: 1 to 6 and 8 to 16 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 6 and 8 to 16, more preferably a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a geranyl pyrophosphate (GPP) C6-methyltransferase, preferably a GPP C6-methyltransferase comprising, or consisting of, SEQ ID NO: 17 to 31, and variants thereof exhibiting GPP C6-methyltransferase activity and having at least 70 %, preferably at least 80%, more preferably at least 90%, 95%, 96%, 97%, 98% or 99%, sequence identity to any of SEQ ID NO: 17 to 31, more preferably a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17.

In a preferred embodiment, the recombinant microbial host cell comprises
- a heterologous nucleic acid encoding a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a GPP C6-methyltransferase comprising, or consisting of, SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17.

In embodiments wherein the recombinant microbial host cell exhibits geranyl pyrophosphate (GPP) C6-methyltransferase activity, said host cell preferably further exhibits farnesyl diphosphate synthase activity and geranylgeranyl diphosphate synthase activity allowing the production of 14-methylgeranylgeranyl diphosphate (14-meGGPP) from 6-meGPP.

As used herein, the term *"farnesyl diphosphate synthase", "FPP synthase"* or *"IspA"* refers to an enzyme that catalyzes the sequential condensation of isopentenyl pyrophosphate (IPP) with the allylic pyrophosphates, dimethylallyl pyrophosphate, and then with the resultant geranylpyrophosphate (GPP) to the ultimate product farnesyl pyrophosphate (FPP) (EC 2.5.1.10, EC 2.5.1.1). This term also refers to an enzyme that catalyzes the condensation of IPP and 6-meGPP to yield 10-methylfarnesyl pyrophosphate (10-meFPP). FPP synthase activity may be assessed by any method known by the skilled person. For example, this activity can be assessed by incubating a purified FPP synthase with IPP and GPP in the presence of Mg²⁺ and the produced FPP can be analyzed by LC-ESI-MS analysis in negative mode (see e.g. Lee et al. Mass Spectrometry Letters, 2011, 2(4), 92-95).

The recombinant microbial host cell of the invention may naturally express a polypeptide exhibiting FPP synthase. In this case, said recombinant host cell may optionally be genetically modified to overexpress this polypeptide or to further express a heterologous polypeptide exhibiting FPP synthase. In embodiments wherein the recombinant microbial host cell does not naturally express a polypeptide exhibiting FPP synthase, said host cell is genetically modified to express a heterologous polypeptide exhibiting FPP synthase.

As used herein, the term *"geranylgeranyl diphosphate synthase", "GGPP synthase"* or *"CrtE"* refers to an enzyme that catalyzes the condensation of FPP and IPP to yield geranylgeranyl diphosphate (GGPP) (EC 2.5.1.29). This term also refers to an enzyme that catalyzes the condensation of IPP and 10-meFPP to yield 14-meGGPP. GGPP synthase activity may be assessed by any method known by the skilled person. For example, this activity can be assessed by incubating a purified GGPP synthase with IPP and FPP in the presence of Mg²⁺, and the produced GGPP can be detected by LC-ESI-MS analysis in negative mode (see e.g. Lee et al. Mass Spectrometry Letters, 2011, 2(4), 92-95).

The recombinant microbial host cell of the invention may naturally express a polypeptide exhibiting GGPP synthase. In this case, said recombinant host cell may optionally be genetically modified to overexpress this polypeptide or to further express a heterologous polypeptide exhibiting GGPP synthase. In embodiments wherein the recombinant microbial host cell does not naturally express a polypeptide exhibiting GGPP synthase, said host cell is genetically modified to express a heterologous polypeptide exhibiting GGPP synthase.

The FPP synthase activity and the GGPP synthase activity of the recombinant microbial host cell of the invention may be provided by two distinct enzymes, i.e. a FPP synthase and a GGPP synthase, or may be provided by only one enzyme exhibiting these two activities. Thus, the recombinant microbial host cell of the invention may comprise (i) a nucleic acid encoding a FPP synthase and/or a nucleic acid encoding a GGPP synthase, and/or (ii) a nucleic acid encoding a polypeptide exhibiting FPP synthase and GGPP synthase activities.

In preferred embodiments, the recombinant microbial host cell comprises a heterologous nucleic acid encoding a GGPP synthase, and optionally a heterologous nucleic acid encoding a FPP synthase.

The FPP synthase may be selected from known FPP synthases and variants thereof exhibiting FPP synthase activity and having at least 70 %, preferably at least 80%, more preferably at least 90%, 95%, 96%, 97%, 98% or 99%, sequence identity to any of said known FPP synthases.

Examples of FPP synthases include, but are not limited to, FPP synthases from E. *coli* (gene: *ispA,* Uniprot accession number: P22939, SEQ ID NO: 32), *Bacillus subtilis* (Uniprot accession number: P54383, SEQ ID NO: 33), *Pantoea agglomerans* (GenBank accession number: UJQ23241.1, SEQ ID NO: 34), *Saccharomyces cerevisiae* (Uniprot accession number: P08524, SEQ ID NO: 35), *Geobacillus stearothermophilus* (Uniprot accession number: Q08291, SEQ ID NO: 36), *Gallus gallus* (Uniprot accession number: P08836, SEQ ID NO: 37) and *Artemisia tridentata* (Uniprot accession number: Q7XYS9, SEQ ID NO: 38). Other FPP synthases can be easily identified using well-known databases or any sequence alignment software applied on the FPP synthases listed above.

Preferably, the FPP synthase comprises, or consists of, an amino acid sequence selected from the group consisting of SEQ ID NO: 32 to 38 and variants thereof exhibiting FPP synthase activity and having at least 70 %, preferably at least 80%, more preferably at least 90%, 95%, 96%, 97%, 98% or 99%, sequence identity to any of SEQ ID NO: 32 to 38. In particular, said variants may comprise, or consist of, a sequence that differs from a sequence set forth in any of SEQ ID No. 32 to 38 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the FPP synthase comprises, or consists of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32. In particular, said variants may comprise, or consist of, a sequence that differs from the sequence of SEQ ID NO. 32 by 1, 2, 3, 4, 5, 6, 7, 8, 9 or 10 substitutions, insertions and/or deletions, preferably by 1, 2, 3, 4 or 5 substitutions, insertions and/or deletions.

The GGPP synthase may be selected from known GGPP synthases and variants thereof exhibiting GGPP synthase activity and having at least 70 %, preferably at least 80%, more preferably at least 90%, 95%, 96%, 97%, 98% or 99%, sequence identity to any of said known GGPP synthases.

Examples of GGPP synthases include, but are not limited to, GGPP synthases from *Pantoea agglomerans* (Uniprot accession number: K7WMD5, SEQ ID NO: 39), *Saccharomyces cerevisiae* (Uniprot accession number: Q12051, SEQ ID NO: 40), *Deinococcus radiodurans* (NCBI accession number: WP_010888034.1, SEQ ID NO : 41), *Synechococcus* sp. (Unitprot accession number: B1XJV9, SEQ ID NO : 42) and *Rhodobacter capsulatus* (Unitprot accession number: P17060, SEQ ID NO : 43). Other GGPP synthases can be easily identified using well-known databases or any sequence alignment software applied on the GGPP synthases listed above.

Preferably, the GGPP synthase comprises, or consists of, an amino acid sequence selected from the group consisting of SEQ ID NO: 39 to 43 and variants thereof exhibiting GGPP synthase activity and having at least 70 %, preferably at least 80%, more preferably at least 90%, 95%, 96%, 97%, 98% or 99%, sequence identity to any of SEQ ID NO: 39 to 43. In particular, said variants may comprise, or consist of, a sequence that differs from a sequence set forth in any of SEQ ID No. 39 to 43 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the GGPP synthase comprises, or consists of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39. In particular, said variants may comprise, or consist of, a sequence that differs from the sequence of SEQ ID NO. 39 by 1, 2, 3, 4, 5, 6, 7, 8, 9 or 10 substitutions, insertions and/or deletions, preferably by 1, 2, 3, 4 or 5 substitutions, insertions and/or deletions.

Examples of polypeptides exhibiting FPP synthase and GGPP synthase activities include, but are not limited to, IdsA from *Methanobacterium thermoautotrophicum* (Uniprot accession number: 026156, SEQ ID NO: 44) and FPP/GGPP synthase from *Pyrococcus furiosus* (Uniprot accession number: Q8U1V3, SEQ ID NO: 45). Other polypeptides exhibiting FPP synthase and GGPP synthase activities can be easily identified using well-known databases or any sequence alignment software.

Preferably, the polypeptide exhibiting FPP synthase and GGPP synthase activities comprises, or consists of, an amino acid sequence selected from the group consisting of SEQ ID NO: 44 to 45 and variants thereof exhibiting FPP synthase and GGPP synthase activities and having at least 70 %, preferably at least 80%, more preferably at least 90%, 95%, 96%, 97%, 98% or 99%, sequence identity to any of SEQ ID NO: 44 to 45. In particular, said variants may comprise, or consist of, a sequence that differs from a sequence set forth in any of SEQ ID No. 44 to 45 by 1, 2, 3, 4, 5, 6, 7, 8, 9 or 10 substitutions, insertions and/or deletions, preferably by 1, 2, 3, 4 or 5 substitutions, insertions and/or deletions.

In a particular embodiment, the recombinant microbial host cell comprises
- a heterologous nucleic acid encoding a lycopene cyclase, preferably a lycopene cyclase comprising, or consisting of, an amino acid sequence selected from the group consisting of SEQ ID NO: 1 to 6 and 8 to 16 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 6 and 8 to 16, more preferably a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a geranyl pyrophosphate (GPP) C6-methyltransferase, preferably a GPP C6-methyltransferase comprising, or consisting of, SEQ ID NO: 17 to 31, and variants thereof exhibiting GPP C6-methyltransferase activity and having at least 70 %, preferably at least 80%, more preferably at least 90%, 95%, 96%, 97%, 98% or 99%, sequence identity to any of SEQ ID NO: 17 to 31, more preferably a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase, preferably a GGPP synthase comprising, or consisting of, an amino acid sequence selected from the group consisting of SEQ ID NO: 39 to 43 and variants thereof exhibiting GGPP synthase activity and having at least 70 %, preferably at least 80%, more preferably at least 90%, 95%, 96%, 97%, 98% or 99%, sequence identity to any of SEQ ID NO: 39 to 43, more preferably a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39.

Optionally, the recombinant microbial host cell may further comprise a heterologous nucleic acid encoding a FPP synthase, preferably a FPP synthase comprising, or consisting of, an amino acid sequence selected from the group consisting of SEQ ID NO: 32 to 38 and variants thereof exhibiting FPP synthase activity and having at least 70 %, preferably at least 80%, more preferably at least 90%, 95%, 96%, 97%, 98% or 99%, sequence identity to any of SEQ ID NO: 32 to 38, more preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32.

In a preferred embodiment, the recombinant microbial host cell comprises
- a heterologous nucleic acid encoding a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39.

Optionally, the recombinant microbial host cell may further comprise a heterologous nucleic acid encoding a FPP synthase, preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32.

### Phytoene synthase activity and phytoene desaturase activity

The recombinant microbial host cell of the invention preferably further exhibits phytoene synthase activity and phytoene desaturase activity allowing the production of 2-methyl-lycopene and/or 2,2'-dimethyl-lycopene from 14-meGGPP, preferably the production of 2-methyl-lycopene and 2,2'-dimethyl-lycopene.

As used herein, the term *"phytoene synthase"* or *"CrtB"* refers to an enzyme that catalyzes the condensation of two molecules of geranylgeranyl diphosphate (GGPP) to yield phytoene (EC 2.5.1.32). This term also refers to an enzyme that catalyzes the condensation of two molecules of 14-meGGPP or one 14-meGGPP and one GGPP to yield 2,2'-dimethyl-phytoene or 2-methyl-phytoene. Phytoene synthase activity may be assessed by any method known by the skilled person. For example, this activity may be assessed by a bacterial complementation assay in *E. coli* wherein a putative *crtB* is coexpressed with a plasmid coding all enzymes to produce carotene except the phytoene synthase (see e.g. Cunningham et al., Photosynth Res, 2007, 92, 245-259). The functionality of CrtB can be detected by color change of *E. coli* colonies to yellow. The subsequent production of carotene can be detected by LC-MS and/or by UV/Vis spectroscopy.

The recombinant microbial host cell of the invention may naturally express a phytoene synthase. In this case, said recombinant host cell may optionally be genetically modified to overexpress this protein or to further express a heterologous phytoene synthase. In embodiments wherein the recombinant microbial host cell does not naturally express a phytoene synthase, said host cell is genetically modified to express a heterologous phytoene synthase.

As used herein, the term *"phytoene desaturase", "CrtI"* refers to an enzyme catalyzing four desaturation steps to convert phytoene to lycopene (EC 1.3.99.31). This term also refers to an enzyme that catalyzes two desaturation steps to convert 2-methyl-phytoene and 2, 2'-dimethyl-phytoene to 2-methyl-lycopene and 2,2'-dimethyl-lycopene, respectively. Phytoene desaturase activity may be assessed by any method known by the skilled person. For example, this activity may be assessed by a bacterial complementation assay in *E. coli* wherein a putative *crtI is* coexpressed with a plasmid coding all enzymes to produce carotene except the phytoene desaturase (see e.g. Cunningham et al., Photosynth Res, 2007, 92, 245-259). The functionality of CrtI can be detected by color change of *E. coli* colonies to pale yellow. The subsequent production of carotene can be detected by LC-MS and/or by UV/Vis spectroscopy.

The recombinant microbial host cell of the invention may naturally express a phytoene desaturase. In this case, said recombinant host cell may optionally be genetically modified to overexpress this protein or to further express a heterologous phytoene desaturase. In embodiments wherein the recombinant microbial host cell does not naturally express a phytoene desaturase, said host cell is genetically modified to express a heterologous phytoene desaturase.

In an embodiment, the recombinant microbial host cell of the invention comprises a heterologous nucleic acid encoding a phytoene synthase.

In another embodiment, the recombinant microbial host cell of the invention comprises a heterologous nucleic acid encoding a lycopene-forming phytoene desaturase.

In a preferred embodiment, the recombinant microbial host cell of the invention comprises a heterologous nucleic acid encoding a phytoene synthase and a heterologous nucleic acid encoding a lycopene-forming phytoene desaturase.

The phytoene synthase may be selected from known phytoene synthases and variants thereof exhibiting phytoene synthase activity and having at least 70 %, preferably at least 80%, more preferably at least 90%, 95%, 96%, 97%, 98% or 99%, sequence identity to any of said known phytoene synthases.

Examples of phytoene synthases include, but are not limited to, phytoene synthases from *Pantoea agglomerans* (Uniprot accession number: D5KXJ0, SEQ ID NO: 46), *Rhodobacter capsulatus* (Uniprot accession number: P17056, SEQ ID NO: 47). Other phytoene synthases can be easily identified using well-known databases or any sequence alignment software applied on the phytoene synthases listed above.

Preferably, the phytoene synthase comprises, or consists of, an amino acid sequence selected from the group consisting of SEQ ID NO: 46 to 47 and variants thereof exhibiting phytoene synthase activity and having at least 70 %, preferably at least 80%, more preferably at least 90%, 95%, 96%, 97%, 98% or 99%, sequence identity to any of SEQ ID NO: 46 to 47. In particular, said variants may comprise, or consist of, a sequence that differs from a sequence set forth in any of SEQ ID No. 46 to 47 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the phytoene synthase comprises, or consists of, an amino acid sequence selected from the group consisting of SEQ ID NO: 46, and variants thereof exhibiting phytoene synthase activity and having at least 70 %, preferably at least 80%, more preferably at least 90%, 95%, 96%, 97%, 98% or 99%, sequence identity to SEQ ID NO: 46. In particular, said variants may comprise, or consist of, a sequence that differs from the sequence of SEQ ID NO. 46 by 1, 2, 3, 4, 5, 6, 7, 8, 9 or 10 substitutions, insertions and/or deletions, preferably by 1, 2, 3, 4 or 5 substitutions, insertions and/or deletions.

The lycopene-forming phytoene desaturase may be selected from known lycopene-forming phytoene desaturase and variants thereof exhibiting lycopene-forming phytoene desaturase activity and having at least 70 %, preferably at least 80%, more preferably at least 90%, 95%, 96%, 97%, 98% or 99%, sequence identity to any of said known lycopene-forming phytoene desaturases.

Examples of lycopene-forming phytoene desaturases include, but are not limited to, lycopene-forming phytoene desaturases from *Pantoea agglomerans* (Uniprot accession number: L0BGV3, SEQ ID NO: 48), *Rhodobacter capsulatus* (Uniprot accession number: A0A4U1JQP1, SEQ ID NO: 49). Other lycopene-forming phytoene desaturases can be easily identified using well-known databases or any sequence alignment software applied on the phytoene desaturases listed above.

Preferably, the lycopene-forming phytoene desaturase comprises, or consists of, an amino acid sequence selected from the group consisting of SEQ ID NO: 48 to 49 and variants thereof exhibiting lycopene-forming phytoene desaturase activity and having at least 70 %, preferably at least 80%, more preferably at least 90%, 95%, 96%, 97%, 98% or 99%, sequence identity to any of SEQ ID NO: 48 to 49. In particular, said variants may comprise, or consist of, a sequence that differs from a sequence set forth in any of SEQ ID No. 48 to 49 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the lycopene-forming phytoene desaturase comprises, or consists of, an amino acid sequence selected from the group consisting of SEQ ID NO: 48, and variants thereof exhibiting lycopene-forming phytoene desaturase activity and having at least 70 %, preferably at least 80%, more preferably at least 90%, 95%, 96%, 97%, 98% or 99%, sequence identity to SEQ ID NO: 48. In particular, said variants may comprise, or consist of, a sequence that differs from the sequence of SEQ ID NO. 48 by 1, 2, 3, 4, 5, 6, 7, 8, 9 or 10 substitutions, insertions and/or deletions, preferably by 1, 2, 3, 4 or 5 substitutions, insertions and/or deletions.

In a particular embodiment, the recombinant microbial host cell comprises
- a heterologous nucleic acid encoding a lycopene cyclase, preferably a lycopene cyclase comprising, or consisting of, an amino acid sequence selected from the group consisting of SEQ ID NO: 1 to 6 and 8 to 16 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 6 and 8 to 16, more preferably a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a geranyl pyrophosphate (GPP) C6-methyltransferase, preferably a GPP C6-methyltransferase comprising, or consisting of, SEQ ID NO: 17 to 31, and variants thereof exhibiting GPP C6-methyltransferase activity and having at least 70 %, preferably at least 80%, more preferably at least 90%, 95%, 96%, 97%, 98% or 99%, sequence identity to any of SEQ ID NO: 17 to 31, more preferably a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase, preferably a GGPP synthase comprising, or consisting of, an amino acid sequence selected from the group consisting of SEQ ID NO: 39 to 43 and variants thereof exhibiting GGPP synthase activity and having at least 70 %, preferably at least 80%, more preferably at least 90%, 95%, 96%, 97%, 98% or 99%, sequence identity to any of SEQ ID NO: 39 to 43, more preferably a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39; and
- a heterologous nucleic acid encoding a phytoene synthase, preferably a phytoene synthase comprising, or consisting of, an amino acid sequence selected from the group consisting of SEQ ID NO: 46 to 47 and variants thereof exhibiting phytoene synthase activity and having at least 70 %, preferably at least 80%, more preferably at least 90%, 95%, 96%, 97%, 98% or 99%, sequence identity to any of SEQ ID NO: 46 to 47, more preferably a phytoene synthase comprising, or consisting of, an amino acid sequence selected from the group consisting of SEQ ID NO: 46, and variants thereof exhibiting phytoene synthase activity and having at least 70 %, preferably at least 80%, more preferably at least 90%, 95%, 96%, 97%, 98% or 99%, sequence identity to SEQ ID NO: 46; and
- a heterologous nucleic acid encoding a lycopene-forming phytoene desaturase, preferably a lycopene-forming phytoene desaturase comprising, or consisting of, an amino acid sequence selected from the group consisting of SEQ ID NO: 48 to 49 and variants thereof exhibiting lycopene-forming phytoene desaturase activity and having at least 70 %, preferably at least 80%, more preferably at least 90%, 95%, 96%, 97%, 98% or 99%, sequence identity to any of SEQ ID NO: 48 to 49, more preferably a lycopene-forming phytoene desaturase comprising, or consisting of, an amino acid sequence selected from the group consisting of SEQ ID NO: 48, and variants thereof exhibiting lycopene-forming phytoene desaturase activity and having at least 70 %, preferably at least 80%, more preferably at least 90%, 95%, 96%, 97%, 98% or 99%, sequence identity to SEQ ID NO: 48.

Optionally, the recombinant microbial host cell further comprises a heterologous nucleic acid encoding a FPP synthase, preferably a FPP synthase comprising, or consisting of, an amino acid sequence selected from the group consisting of SEQ ID NO: 32 to 38 and variants thereof exhibiting FPP synthase activity and having at least 70 %, preferably at least 80%, more preferably at least 90%, 95%, 96%, 97%, 98% or 99%, sequence identity to any of SEQ ID NO: 32 to 38, more preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32

In a preferred embodiment, the recombinant microbial host cell comprises
- a heterologous nucleic acid encoding a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39; and
- a heterologous nucleic acid encoding a phytoene synthase comprising, or consisting of, an amino acid sequence selected from the group consisting of SEQ ID NO: 46, and variants thereof exhibiting phytoene synthase activity and having at least 70 %, preferably at least 80%, more preferably at least 90%, 95%, 96%, 97%, 98% or 99%, sequence identity to SEQ ID NO: 46; and
- a heterologous nucleic acid encoding a lycopene-forming phytoene desaturase comprising, or consisting of, an amino acid sequence selected from the group consisting of SEQ ID NO: 48, and variants thereof exhibiting lycopene-forming phytoene desaturase activity and having at least 70 %, preferably at least 80%, more preferably at least 90%, 95%, 96%, 97%, 98% or 99%, sequence identity to SEQ ID NO: 48.

Optionally, the recombinant microbial host cell may further comprise a heterologous nucleic acid encoding a FPP synthase, preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32.

### Carotenoid cleavage dioxygenase activity

The recombinant microbial host cell of the invention preferably further exhibits carotenoid cleavage dioxygenase activity allowing the production of irone compounds, in particular β- and/or α-irones, from 2-methyl-carotene and/or 2,2'-dimethyl-carotene.

As used herein, the term *"carotenoid cleavage dioxygenase"* or *"CCD"* refers to an enzyme that catalyzes the formation of apocarotenoids by double-bond cleavage of cyclic carotenoids. Preferably, this term refers to a CCD class 1 enzyme (CCD1), i.e. an enzyme that cleaves a variety of carotenoids symmetrically at both the 9-10 and 9'-10' double bonds. In particular, the CDD enzyme used in the present invention catalyzes the cleavage of the double bonds between C9-C10 and C9'-C10' of 2-methyl-carotene and/or 2,2'-dimethyl-carotene. CCD activity, and in particular CCD1 activity, may be assessed by any method known by the skilled person. For example, this activity may be assessed by incubating acetone extracts of cells producing carotene substrates and purified CCD1 in the presence of β-octylglucoside, followed by analyzing the cleaved products by LC-ESI-MS.

The recombinant microbial host cell of the invention may naturally express a CCD. In this case, said recombinant host cell may optionally be genetically modified to overexpress this protein or to further express a heterologous CCD. In embodiments wherein the recombinant microbial host cell does not naturally express a CCD, said host cell is genetically modified to express a heterologous CCD.

In preferred embodiments, the recombinant microbial host cell of the invention comprises a heterologous nucleic acid encoding a CCD.

The CCD may be selected from known CCDs and variants thereof exhibiting CCD activity and having at least 70 %, preferably at least 80%, more preferably at least 90%, 95%, 96%, 97%, 98% or 99%, sequence identity to any of said known CCDs.

Examples of CCDs include, but are not limited to, CCDs from *Osmanthus fragrans* (CCD1_{OF}, NCBI GenBank accession number: BAJ05401.1, SEQ ID NO: 50), *Zea mays* (GenBank accession number: ABF85668.1, SEQ ID NO: 51), *Arabidopsis thaliana* (Uniprot accession number: O65572, SEQ ID NO: 52), *Solanum lycopersicum* (Uniprot accession number: Q6E4P5, SEQ ID NO: 53) and *Chrysanthemum morifolium* (Uniprot accession number: A0JBX5, SEQ ID NO: 54). Other CCDs can be easily identified using well-known databases or any sequence alignment software applied on the CCDs listed above.

Preferably, the CCD comprises, or consists of, an amino acid sequence selected from the group consisting of SEQ ID NO: 50 to 54 and variants thereof exhibiting CCD activity and having at least 70 %, preferably at least 80%, more preferably at least 90%, 95%, 96%, 97%, 98% or 99%, sequence identity to any of SEQ ID NO: 50 to 54. In particular, said variants may comprise, or consist of, a sequence that differs from a sequence set forth in any of SEQ ID No. 50 to 54 by 1, 2, 3, 4, 5, 6, 7, 8, 9 or 10 substitutions, insertions and/or deletions, preferably by 1, 2, 3, 4 or 5 substitutions, insertions and/or deletions.

More preferably, the CCD comprises, or consists of, an amino acid sequence selected from the group consisting of SEQ ID NO: 50 and variants thereof exhibiting CCD activity and having at least 70 %, preferably at least 80%, more preferably at least 90%, 95%, 96%, 97%, 98% or 99%, sequence identity to SEQ ID NO: 50. In particular, said variants may comprise, or consist of, a sequence that differs from the sequence of SEQ ID NO. 50 by 1, 2, 3, 4, 5, 6, 7, 8, 9 or 10 substitutions, insertions and/or deletions, preferably by 1, 2, 3, 4 or 5 substitutions, insertions and/or deletions.

In a particular embodiment, the recombinant microbial host cell comprises
- a heterologous nucleic acid encoding a lycopene cyclase, preferably a lycopene cyclase comprising, or consisting of, an amino acid sequence selected from the group consisting of SEQ ID NO: 1 to 6 and 8 to 16 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 6 and 8 to 16, more preferably a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a geranyl pyrophosphate (GPP) C6-methyltransferase, preferably a GPP C6-methyltransferase comprising, or consisting of, SEQ ID NO: 17 to 31, and variants thereof exhibiting GPP C6-methyltransferase activity and having at least 70 %, preferably at least 80%, more preferably at least 90%, 95%, 96%, 97%, 98% or 99%, sequence identity to any of SEQ ID NO: 17 to 31, more preferably a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase, preferably a GGPP synthase comprising, or consisting of, an amino acid sequence selected from the group consisting of SEQ ID NO: 39 to 43 and variants thereof exhibiting GGPP synthase activity and having at least 70 %, preferably at least 80%, more preferably at least 90%, 95%, 96%, 97%, 98% or 99%, sequence identity to any of SEQ ID NO: 39 to 43, more preferably a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39; and
- a heterologous nucleic acid encoding a phytoene synthase, preferably a phytoene synthase comprising, or consisting of, an amino acid sequence selected from the group consisting of SEQ ID NO: 46 to 47 and variants thereof exhibiting phytoene synthase activity and having at least 70 %, preferably at least 80%, more preferably at least 90%, 95%, 96%, 97%, 98% or 99%, sequence identity to any of SEQ ID NO: 46 to 47, more preferably a phytoene synthase comprising, or consisting of, an amino acid sequence selected from the group consisting of SEQ ID NO: 46, and variants thereof exhibiting phytoene synthase activity and having at least 70 %, preferably at least 80%, more preferably at least 90%, 95%, 96%, 97%, 98% or 99%, sequence identity to SEQ ID NO: 46; and
- a heterologous nucleic acid encoding a lycopene-forming phytoene desaturase, preferably a lycopene-forming phytoene desaturase comprising, or consisting of, an amino acid sequence selected from the group consisting of SEQ ID NO: 48 to 49 and variants thereof exhibiting lycopene-forming phytoene desaturase activity and having at least 70 %, preferably at least 80%, more preferably at least 90%, 95%, 96%, 97%, 98% or 99%, sequence identity to any of SEQ ID NO: 48 to 49, more preferably a lycopene-forming phytoene desaturase comprising, or consisting of, an amino acid sequence selected from the group consisting of SEQ ID NO: 48, and variants thereof exhibiting lycopene-forming phytoene desaturase activity and having at least 70 %, preferably at least 80%, more preferably at least 90%, 95%, 96%, 97%, 98% or 99%, sequence identity to SEQ ID NO: 48; and
a heterologous nucleic acid encoding a CCD, preferably a CCD comprising, or consisting of, an amino acid sequence selected from the group consisting of SEQ ID NO: 50 to 54 and variants thereof exhibiting CCD activity and having at least 70 %, preferably at least 80%, more preferably at least 90%, 95%, 96%, 97%, 98% or 99%, sequence identity to any of SEQ ID NO: 50 to 54, more preferably a CCD comprising, or consisting of, an amino acid sequence selected from the group consisting of SEQ ID NO: 50, and variants thereof exhibiting CCD activity and having at least 70 %, preferably at least 80%, more preferably at least 90%, 95%, 96%, 97%, 98% or 99%, sequence identity to SEQ ID NO: 50.

Optionally, the recombinant microbial host cell further comprises a heterologous nucleic acid encoding a FPP synthase, preferably a FPP synthase comprising, or consisting of, an amino acid sequence selected from the group consisting of SEQ ID NO: 32 to 38 and variants thereof exhibiting FPP synthase activity and having at least 70 %, preferably at least 80%, more preferably at least 90%, 95%, 96%, 97%, 98% or 99%, sequence identity to any of SEQ ID NO: 32 to 38, more preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32

In a preferred embodiment, the recombinant microbial host cell comprises
- a heterologous nucleic acid encoding a lycopene cyclase comprising, or consisting of, an amino acid sequence selected from the group consisting of SEQ ID NO: 1 to 3 and 8 to 11 and variants thereof exhibiting lycopene cyclase activity and having at least 70 %, preferably at least 80%, more preferably at least 90%, 95%, 96%, 97%, 98% or 99%, sequence identity to any of SEQ ID NO: 1 to 3 and 8 to 11; and
- a heterologous nucleic acid encoding a GPP C6-methyltransferase comprising, or consisting of, an amino acid sequence selected from the group consisting of SEQ ID NO: 17 or 18, preferably SEQ ID NO: 17, and variants thereof exhibiting GPP C6-methyltransferase activity and having at least 70 %, preferably at least 80%, more preferably at least 90%, 95%, 96%, 97%, 98% or 99%, sequence identity to SEQ ID NO: 17 or 18, preferably SEQ ID NO: 17; and
- a heterologous nucleic acid encoding a GGPP synthase comprising, or consisting of, an amino acid sequence selected from the group consisting of SEQ ID NO: 39, and variants thereof exhibiting GGPP synthase activity and having at least 70 %, preferably at least 80%, more preferably at least 90%, 95%, 96%, 97%, 98% or 99%, sequence identity to SEQ ID NO: 39; and
- a heterologous nucleic acid encoding a phytoene synthase comprising, or consisting of, an amino acid sequence selected from the group consisting of SEQ ID NO: 46, and variants thereof exhibiting phytoene synthase activity and having at least 70 %, preferably at least 80%, more preferably at least 90%, 95%, 96%, 97%, 98% or 99%, sequence identity to SEQ ID NO: 46; and
- a heterologous nucleic acid encoding a lycopene-forming phytoene desaturase comprising, or consisting of, an amino acid sequence selected from the group consisting of SEQ ID NO: 48, and variants thereof exhibiting lycopene-forming phytoene desaturase activity and having at least 70 %, preferably at least 80%, more preferably at least 90%, 95%, 96%, 97%, 98% or 99%, sequence identity to SEQ ID NO: 48; and
- a heterologous nucleic acid encoding a CCD comprising, or consisting of, an amino acid sequence selected from the group consisting of SEQ ID NO: 50, and variants thereof exhibiting CCD activity and having at least 70 %, preferably at least 80%, more preferably at least 90%, 95%, 96%, 97%, 98% or 99%, sequence identity to SEQ ID NO: 50.

Optionally, the recombinant microbial host cell may further comprise a heterologous nucleic acid encoding a FPP synthase, preferably a FPP synthase comprising, or consisting of, an amino acid sequence selected from the group consisting of SEQ ID NO: 32, and variants thereof exhibiting FPP synthase activity and having at least 70 %, preferably at least 80%, more preferably at least 90%, 95%, 96%, 97%, 98% or 99%, sequence identity to SEQ ID NO: 32.

In preferred embodiments, the recombinant microbial host cell of the invention exhibits (i) lycopene β-cyclase and/or lycopene ε-cyclase activities and (ii) GPP C6-methyltransferase, FPP synthase, GGPP synthase, phytoene synthase and lycopene-forming phytoene desaturase activities. Optionally, the recombinant microbial host cell of the invention may further exhibit CCD activity. Each of these activities may be provided by heterologous or endogenous enzymes.

### Inhibition of phosphatases degrading phosphorylated precursors

It was previously shown that inhibition of PgpB and CpdB encoding phosphatases that degrade phosphorylated isoprene precursors results in an enhanced carotenoid accumulation in *E. coli* due to the increased availability of terpene precursors (e.g., GPP and FPP) (Wang et al., Appl. Microbiol. Biotechnol. 102 (2018) 9771-9780). These phosphatases are also capable of degrading 13-meFPP to produce 13-methylfarnesyl.

Thus, the recombinant microbial host cell may be further genetically modified to reduce the activity of an endogenous PgpB phosphatase and/or the activity of an endogenous CpdB phosphatase by comparison to the non-modified microbial host cell. Preferably, the recombinant microbial host cell is genetically modified to reduce the activity of an endogenous PgpB phosphatase and the activity of an endogenous CpdB phosphatase. In preferred embodiments, the activity of an endogenous PgpB phosphatase and/or the activity of an endogenous CpdB phosphatase are suppressed.

As used herein the term *"PgpB", "PgpB phosphatase"* or *"PgpB encoding phosphatase"* refers to a phosphatidylglycerophosphatase B that catalyzes the dephosphorylation of diacylglycerol diphosphate (DGPP) to phosphatidate (PA) and the subsequent dephosphorylation of PA to diacylglycerol (DAG). In *E. coli,* this enzyme is encoded by the gene *pgpB.*

As used herein the term *"CpdB", "CpdB phosphatase"* or *"CpdB encoding phosphatase"* refers to a 2',3'-cyclic-nucleotide 2'-phosphodiesterase/3'-nucleotidase that converts a 2',3'-cyclic nucleotide to a 3'-nucleotide and then the 3'-nucleotide to the corresponding nucleoside and phosphate. In *E. coli,* this enzyme is encoded by the gene *cpdB.*

According to the organism, the nomenclature of these enzymes and encoding genes may vary. However, for the sake of clarity, in the present specification, these terms are used independently from the origin of the enzymes or genes.

The activity of PgpB phosphatase may be assessed using any method known by the skilled person. For example, said activity may be assessed by incubating suitable lipid phosphate substrates, such as lysophosphatidic acid, with purified PgpB protein in the presence of n-dodecyl-β-d-maltoside. The released phosphate can be detected by a colorimetric assay as described in Tong et al. (J Biol Chem. 2016 Aug 26; 291(35): 18342-18352).

The activity of CpdB phosphatase may be assessed using any method known by the skilled person. For example, said activity may be assessed by incubating purified CpdB protein with cyclic-dinucleotide phosphate substrates such as c-di-AMP. The resulting nucleotide product can be detected by HPLC coupled to UV detection or LC-MS.

Examples of PgpB phosphatases include, but are not limited to, PgpB phosphatases from *E. coli* (e.g. Uniprot accession number: P0A924, SEQ ID NO: 55) and *Bacillus subtilis* (e.g. Uniprot accession number: O34349, SEQ ID NO: 56).

Examples of CpdB phosphatases include, but are not limited to, CpdB phosphatases from *E. coli* (e.g. Uniprot accession number: P08331, SEQ ID NO: 57) and *Salmonella typhimurium* (e.g. Uniprot accession number: P26265, SEQ ID NO: 58).

The gene encoding endogenous PgpB or CpdB phosphatase in a host cell can be easily identified using well-known method for the skilled person, e.g. using well-known databases or any sequence alignment software applied on known PgpB or CpdB phosphatases as listed above.

The endogenous PgpB or CpdB activity may be reduced or suppressed using any method known by the skilled person. In particular, this activity may be reduced by reducing the expression of the endogenous gene, for example, by replacing the endogenous promoter by a weaker promoter. Preferably, the gene encoding the endogenous PgpB or CpdB is inactivated by any method known by the skilled person, for example by deletion of all or part of this gene, by introducing a nonsense codon, a cassette, a gene or a mutation inducing a frameshift. In a preferred embodiment, the gene encoding the endogenous PgpB and/or the gene encoding the endogenous CpdB are inactivated by deleting all or part of said gene(s). More preferably, the genes encoding the endogenous PgpB and CpdB are inactivated by deleting all or part of said genes.

### Improvement of SAM pool

S-Adenosylmethionine (SAM) is a ubiquitous intracellular methyl donor. As a methyl donor, SAM normally donates its methyl group in enzyme-catalyzed reactions, such as methylation of GPP by a GPP C6-methyltransferase to produce 6-meGPP. SAM is synthesized from methionine and ATP by SAM synthetase, encoded in *E. coli* by the *metK* gene. MetJ is a repressor protein which controls expression of the genes involved in methionine biosynthesis and transport in *E. coli.* In particular, when combined with SAM, MetJ represses the expression of the methionine regulon and of enzymes involved in SAM synthesis.

Thus, the recombinant microbial host cell may be further genetically modified to reduce the activity of an endogenous MetJ repressor by comparison to the non-modified microbial host cell. Preferably, the recombinant microbial host cell is genetically modified to suppress the activity of an endogenous MetJ repressor.

As used herein the term *"MetJ", "MetJ protein"* or *"MetJ repressor"* refers to a Met repressor that is the ligand-responsive transcriptional repressor that naturally regulates production of methionine and SAM in response to feedback from SAM accumulation (Cress et al., Microb. Cell Fact. 16 (2017) 1-14). In *E. coli,* this enzyme is encoded by the gene *metJ.* According to the organism, the nomenclature of this protein and encoding gene may vary. However, for the sake of clarity, in the present specification, these terms are used independently from the origin of the protein or gene.

The activity of MetJ repressor may be assessed using any method known by the skilled person. For example, said activity may be assessed by studying the interaction of MetJ with consensus DNA sequences, in the presence or absence of SAM, by gel shift assays and/or analytical ultracentrifugation (Augustus et al. Biochemistry 2010, 49, 15, 3289-3295).

Examples of MetJ repressors include, but are not limited to, MetJ repressors from *E. coli* (e.g. Uniprot accession number: P0A8U6, SEQ ID NO: 59) and *Salmonella typhimurium* (e.g. Uniprot accession number: P06203, SEQ ID NO: 60).

The gene encoding endogenous MetJ repressor in a host cell can be easily identified using well-known method for the skilled person, e.g. using well-known databases or any sequence alignment software applied on known MetJ repressors as listed above.

The endogenous MetJ activity may be reduced or suppressed using any method known by the skilled person. In particular, this activity may be reduced by reducing the expression of the endogenous gene, for example, by replacing the endogenous promoter by a weaker promoter. Preferably, the gene encoding the endogenous MetJ is inactivated by any method known by the skilled person, for example by deletion of all or part of this gene, by introducing a nonsense codon, a cassette, a gene or a mutation inducing a frameshift. In a preferred embodiment, the gene encoding the endogenous MetJ is inactivated by deleting all or part of said gene.

### Increasing the pool of DMAPP and IPP precursors

GPP, the substrate of the GPP C6-methyltransferase, is synthesized by a FPP synthase that catalyzes the sequential condensation of isopentenyl pyrophosphate (IPP) with dimethylallyl pyrophosphate (DMAPP) to produce GPP. IPP and its isomer DMAPP are synthesized via the mevalonate pathway (MVA) or via the non-mevalonate pathway (or 2-C-methyl-D-erythritol 4-phosphate/1-deoxy-D-xylulose 5-phosphate (MEP/DXP) pathway). MVA pathway is utilized by most eukaryotes as well as archaebacteria, while the MEP pathway is found in most bacteria. Some exception occurs, for example *Staphylococcus aureus* uses the MVA pathway.

The recombinant microbial host cell of the invention may be genetically modified to increase the pool of DMAPP and IPP by
(i) introducing a heterologous MVA pathway or overexpressing one or several enzymes of the MVA pathway, and/or
(ii) introducing a heterologous MEP pathway or overexpressing one or several enzymes of the MEP pathway.

Preferably, in embodiments wherein the recombinant host cell of the invention comprises an endogenous MVA pathway, said host cell may be genetically modified by introducing a heterologous MEP pathway or overexpressing one or several enzymes of the MVA pathway. On the other hand, in embodiments wherein the recombinant host cell of the invention comprises an endogenous MEP pathway, said host cell is preferably genetically modified by introducing a heterologous MVA pathway or overexpressing one or several enzymes of the MEP pathway.

Enzymes of the MVA or MEP pathway to be overexpressed may be endogenous or heterologous enzymes.

In an embodiment, the recombinant microbial host cell of the invention has been genetically modified to introduce a heterologous MVA pathway or to overexpress one or several enzymes of the MVA pathway. Preferably, the recombinant microbial host cell of the invention has been genetically modified to introduce a heterologous MVA pathway.

The term "mevalonate pathway" or "MVA pathway" is used herein to refer to the biosynthetic pathway that converts acetyl-CoA to IPP. The mevalonate pathway comprises enzymes that catalyze the following steps: (a) condensing two molecules of acetyl-CoA to acetoacetyl-CoA by action of acetyl-CoA C-acetyltransferase (EC 2.3.1.9); (b) condensing acetoacetyl-CoA with acetyl-CoA to form hydroxymethylglutaryl-CoenzymeA (HMG-CoA) by action of hydroxymethylglutaryl-CoA synthase (EC 2.3.3.10); (c) converting HMG-CoA to mevalonate by action of hydroxymethylglutaryl-CoA reductase (NADPH) (EC 1.1.1.34); (d) phosphorylating mevalonate to mevalonate 5-phosphate by action of mevalonate kinase (EC 2.7.1.36); (e) converting mevalonate 5-phosphate to mevalonate 5-pyrophosphate by action of phosphomevalonate kinase (EC 2.7.4.2); and (f) converting mevalonate 5-pyrophosphate to isopentenyl pyrophosphate by action of diphosphomevalonate decarboxylase (EC 4.1.1.33).

Genes encoding enzymes of a MVA pathway, to be heterologously expressed or overexpressed, may be easily identified by the skilled person. In particular, heterologous genes may be obtained from various sources including plants, fungi and yeasts, in particular from *Saccharomyces cerevisiae.* As example, the MVA pathway of *Saccharomyces cerevisiae* comprises hydroxymethylglutaryl-CoA synthase encoded by the gene *erg13* (Uniprot accession number: P54839), hydroxymethylglutaryl-CoA reductase (NADPH) encoded by the gene *hmg1* (Uniprot accession number: P12683), mevalonate kinase encoded by the gene *erg12* (Uniprot accession number: P07277), phosphomevalonate kinase encoded by the gene *erg8* (Uniprot accession number: P24521), and diphosphomevalonate decarboxylase encoded by the gene *mvd1* (Uniprot accession number: P32377).

In a particular embodiment, the recombinant host cell is *E. coli* and is genetically modified by introducing a heterologous MVA pathway.

In another embodiment, the recombinant microbial host cell of the invention has been genetically modified to introduce a heterologous MEP pathway or to overexpress one or several enzymes of the MEP pathway. Preferably, the recombinant microbial host cell of the invention has been genetically modified to overexpress one or several enzymes of the MEP pathway.

The term "MEP pathway", "MEP/DXP pathway", "non-mevalonate pathway" or "2-C-methyl-D-erythritol 4-phosphate/1-deoxy-D-xylulose 5-phosphate pathway" as used herein refers to the biosynthetic pathway leading to the formation of IPP and DMAPP from the condensation of pyruvate and D-glyceraldehyde 3-phosphate to 1-deoxy-D-xylulose 5- phosphate (DXP). This pathway involves the following enzymes: 1-deoxy-D-xylulose 5- phosphate synthase (EC 2.2.1.7), 1-deoxy-D-xylulose 5-phosphate reductoisomerase (EC 1.1.1.267), 2-C-methyl-D-erythritol 4-phosphate cytidylyltransferase (EC 2.7.7.60), 4-diphosphocytidyl-2-C-methyl-D-erythritol kinase (EC 2.7.1.148), 2-C-methyl-D-erythritol 2,4-cyclodiphosphate synthase (EC 4.6.1.12), 4-hydroxy-3-methylbut-2-en-1-yl diphosphate synthase (EC 1.17.7.1), 4-hydroxy-3-methylbut-2-enyl diphosphate reductase (EC 1.17.1.2), and isopentenyl-diphosphate delta-isomerase (EC 5.3.3.2).

Genes encoding enzymes of a MEP pathway, to be expressed or overexpressed, may be easily identified by the skilled person. As example, the MEP pathway of *E. coli* comprises 1-deoxy-D-xylulose 5- phosphate synthase encoded by the gene *dxs* (Uniprot accession number: P77488), 1-deoxy-D-xylulose 5-phosphate reductoisomerase encoded by the gene *dxr* (Uniprot accession number: P45568), 2-C-methyl-D-erythritol 4-phosphate cytidylyltransferase encoded by the gene *ispD* (Uniprot accession number: Q46893), 4-diphosphocytidyl-2-C-methyl-D-erythritol kinase encoded by the gene *ispE* (Uniprot accession number: P62615), 2-C-methyl-D-erythritol 2,4-cyclodiphosphate synthase encoded by the gene *ispF* (Uniprot accession number: P62617), 4-hydroxy-3-methylbut-2-en-1-yl diphosphate synthase encoded by the gene *ispG* (Uniprot accession number: P62620), 4-hydroxy-3-methylbut-2-enyl diphosphate reductase encoded by the gene *ispH* (Uniprot accession number: P62623) and isopentenyl-diphosphate delta-isomerase encoded by the gene *idi* (Uniprot accession number: Q46822).

In a particular embodiment, the recombinant host cell is *E. coli* and is genetically modified to overexpress one or several enzymes of the MEP pathway, preferably to overexpress at least an *idi* gene.

### Recombinant nucleic acid and expression cassette

Each heterologous nucleic acids as described above is included in an expression cassette. Preferably, the coding nucleic acid sequences have been optimized for expression in the microbial host cell. A coding nucleic acid sequence is operatively linked to the elements required for the expression of the gene, notably for transcription and translation. These elements are chosen so as to be functional in the recombinant microbial host cell. These elements may include, for example, transcription promoters, transcription activators, terminator sequences, and start and stop codons. The methods for selecting these elements as a function of the host cell in which expression is desired are well known to those skilled in the art.

In particular the present invention also relates to an expression cassette useful in the present invention, i.e. an expression cassette comprising at least one nucleic acid selected from the group consisting of a nucleic acid encoding a lycopene cyclase, a nucleic acid encoding a GPP C6-methyltransferase, a nucleic acid encoding a FPP synthase, a nucleic acid encoding a GGPP synthase, a nucleic acid encoding a phytoene synthase, a nucleic acid encoding a lycopene-forming phytoene desaturase and nucleic acid encoding a carotenoid cleavage dioxygenase, and combinations thereof. Preferably, the expression cassette of the invention comprises at least one nucleic acid encoding a lycopene cyclase and at least one nucleic acid selected from the group consisting of a nucleic acid encoding a GPP C6-methyltransferase, a nucleic acid encoding a FPP synthase, a nucleic acid encoding a GGPP synthase, a nucleic acid encoding a phytoene synthase, a nucleic acid encoding a lycopene-forming phytoene desaturase and a nucleic acid encoding a carotenoid cleavage dioxygenase, and combinations thereof. Combinations of heterologous nucleic acids disclosed in the section related to the recombinant microbial host cell of the invention are also contemplated in this aspect. Said nucleic acids are operably linked to one or more control sequences, typically comprising a transcriptional promoter and a transcription terminator, that direct the expression of said nucleic acids.

Each expression cassette may comprise only one coding nucleic acid operably linked to one or more control sequences. Alternatively, expression cassettes useful in the present invention may comprise several coding nucleic acid operably linked to one or more control sequences.

The control sequence typically includes a promoter that is recognized by the host cell. The promoter contains transcriptional control sequences that mediate the expression of the coding nucleic acid. The promoter may be any polynucleotide that shows transcriptional activity in the host cell. The promoter may be a native or heterologous promoter. The promoter may be constitutive or inducible, strong or weak.

Preferably, nucleic acids encoding lycopene cyclase, GPP C6-methyltransferase, FPP synthase, GGPP synthase, phytoene synthase and lycopene-forming phytoene desaturase are placed under the control of one or several constitutive promoter. Preferably, nucleic acid encoding carotenoid cleavage dioxygenase is placed under the control of an inducible promoter

For example, if the microbial host cell is prokaryotic, the promoter may be selected from the following promoters: SJM915, Lacl, LacZ, pLacT, ptac, pARA, pBAD, the RNA polymerase promoters of bacteriophage T3 or T7, the polyhedrin promoter, the PR or PL promoter of lambda phage. In a preferred embodiment, the promoter is SJM915. If the microbial host cell is eukaryotic and in particular a yeast, the promoter may be selected from the following promoters: the promoter pTDH3, the promoter pTEF1, the promoter pTEF2, the promoter pCCW12, the promoter pHHF2, the promoter pHTB2 and the promoter pRPL18B. Examples of inducible promoters that may be used in yeast are the promoters tetO-2, GAL10, GAL10-CYC1 and PHO5.

Optionally, the expression cassette of the invention may also comprise a selectable marker that permits easy selection of recombinant bacteria. Typically, the selectable marker is a gene encoding antibiotic resistance or conferring autotrophy.

The expression cassettes of the invention may be integrated into the genome of the host cell and/or may be maintained in an episomal form into an expression vector. Preferably, the expression cassettes are integrated into the genome of the host cell.

All or part of the expression cassettes comprising the heterologous nucleic acid as described above or a combination of some of them may be included in a common expression vector or in different expression vectors.

The present invention also relates to an expression vector comprising one or several expression cassettes according to the invention.

In particular, the expression vector of the invention may comprise at least one nucleic acid selected from the group consisting of a nucleic acid encoding a lycopene cyclase, a nucleic acid encoding a GPP C6-methyltransferase ,a nucleic acid encoding a FPP synthase, a nucleic acid encoding a GGPP synthase, a nucleic acid encoding a phytoene synthase, a nucleic acid encoding a lycopene-forming phytoene desaturase and a nucleic acid encoding a carotenoid cleavage dioxygenase, and combinations thereof. Preferably, the expression vector of the invention comprises at least one nucleic acid encoding a lycopene cyclase and at least one nucleic acid selected from the group consisting of a nucleic acid encoding a GPP C6-methyltransferase, a nucleic acid encoding a FPP synthase, a nucleic acid encoding a GGPP synthase, a nucleic acid encoding a phytoene synthase, a nucleic acid encoding a lycopene-forming phytoene desaturase and a nucleic acid encoding a carotenoid cleavage dioxygenase, and combinations thereof. Combinations of heterologous nucleic acids disclosed in the section related to the recombinant microbial host cell of the invention are also contemplated in this aspect.

Said expression vector may be used to transform a host cell and enable the expression of the nucleic acid of interest in said cell. The choice of the vector will typically depend on the compatibility of the vector with the host cell into which the vector is to be introduced. The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the host cell, is integrated into the genome and replicated together with the chromosome(s) into which it has been integrated.

The vector preferably comprises one or more selectable markers that permit easy selection of host cells comprising the vector. A selectable marker is a gene the product of which provides for biocide or viral resistance, resistance to heavy metals, prototrophy to auxotrophy, and the like.

The vector preferably comprises an element that permits integration of the vector into the host cell's genome or autonomous replication of the vector in the cell independent of the genome. When integration into the host cell genome occurs, integration of the sequences into the genome may rely on homologous or non-homologous recombination. In one hand, the vector may contain additional polynucleotides for directing integration by homologous recombination at a precise location into the genome of the host cell. These additional polynucleotides may be any sequence that is homologous with the target sequence in the genome of the host cell. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

The methods for selecting these elements according to the host cell in which expression is desired, are well known to one of skill in the art. The vectors may be constructed by the classical techniques of molecular biology, well known to one of skill in the art.

The present invention further relates to the use of an expression cassette or an expression vector according to the invention to transform, transfect or transduce a microbial host cell. The microbial host cell is as defined above.

The host cell may be transformed, transfected or transduced in a transient or stable manner. An expression cassette or vector of the invention is introduced into a host cell so that the cassette or vector is maintained as a chromosomal integrant or as a self-replicating extra-chromosomal vector as described earlier.

The expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation,

Optionally, more than one copy of an expression cassette or expression vector of the present invention may be inserted into the host cell.

The present invention also relates to a method for preparing a recombinant microbial host cell of the invention, comprising the introduction of an expression cassette or expression vector of the invention into the microbial host cell and the selection of microbial host cells comprising said cassette or vector.

### Methods of producing irone compounds

The present invention also relates to a method of producing β- and/or α-irones comprising culturing a recombinant microbial host cell according to the invention, under conditions suitable to produce said β- and/or α-irones and optionally recovering said β- and/or α-irones. It also relates to the use of a recombinant microbial host cell according to the invention to produce β- and/or α-irones.

In embodiments in which the production of β-irones is sought, the recombinant microbial host cell preferably comprises a lycopene cyclase selected from the group consisting of lycopene β-cyclases and lycopene ε-cyclases exhibiting lycopene β-cyclase activity, preferably selected from the group consisting of wild-type bacterial CrtY-type lycopene β-cyclases, wild-type heterodimeric-type lycopene β-cyclases of Gram positive bacteria, and wild-type cyanobacterial CrtL-type lycopene ε-cyclases exhibiting lycopene β-cyclase activity, and the variants thereof exhibiting lycopene β-cyclase activity and having at least 70 %, preferably at least 80%, more preferably at least 90%, 95%, 96%, 97%, 98% or 99%, sequence identity to any of these cyclases. More preferably, the lycopene cyclase comprises, or consists of, an amino acid sequence selected from the group consisting of SEQ ID NO: 1 to 6, 8 and 9, and the variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 6, 8 and 9. Even more preferably, the lycopene cyclase comprises, or consists of, an amino acid sequence selected from the group consisting of SEQ ID NO: 1 to 3, 8 and 9, and the variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 3, 8 and 9.

In embodiments in which the production of α-irones is sought, the recombinant microbial host cell preferably comprises a lycopene cyclase selected from the group consisting of lycopene ε-cyclases, preferably from the group consisting of wild-type cyanobacterial CrtL-type lycopene ε-cyclases, and the variants thereof exhibiting lycopene ε-cyclase activity and having at least 70 %, preferably at least 80%, more preferably at least 90%, 95%, 96%, 97%, 98% or 99%, sequence identity to any of these cyclases. Preferably, the lycopene cyclase comprises, or consists of, an amino acid sequence selected from the group consisting of SEQ ID NO: 8 to 16, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 8 to 16. More preferably, the lycopene cyclase comprises, or consists of, an amino acid sequence selected from the group consisting of SEQ ID NO: 8 to 11, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 8 to 11. Even more preferably, the lycopene cyclase comprises, or consists of, an amino acid sequence selected from the group consisting of SEQ ID NO: 10 and 11, and the variants thereof exhibiting lycopene ε-cyclase activity and having at least 70 %, preferably at least 80%, more preferably at least 90%, 95%, 96%, 97%, 98% or 99%, sequence identity to any of SEQ ID NO: 10 and 11.

In embodiments in which the production of cis-α-irones is sought, the recombinant microbial host cell preferably comprises a lycopene cyclase comprising, or consisting of, an amino acid sequence selected from the group consisting of SEQ ID NO: 10, and the variants thereof exhibiting lycopene ε-cyclase activity and having at least 70 %, preferably at least 80%, more preferably at least 90%, 95%, 96%, 97%, 98% or 99%, sequence identity to SEQ ID NO: 10.

The recombinant microbial host cell may further exhibit GPP C6-methyltransferase activity, FPP synthase activity, GGPP synthase activity, phytoene synthase activity, lycopene-forming phytoene desaturase activity and/or carotenoid cleavage dioxygenase activity as described above. Preferably, the recombinant microbial host cell further exhibits at least GPP C6-methyltransferase activity, FPP synthase activity, GGPP synthase activity, phytoene synthase activity and lycopene-forming phytoene desaturase activity.

In embodiments wherein the recombinant microbial host cell does not exhibit CCD activity, the cleavage of 2-methyl-carotene and/or 2,2'-dimethyl-carotene may be carried out *in vitro,* as illustrated in the experimental section.

The conditions for cultivating the recombinant microbial host cell according to the invention may be adapted according to the conventional techniques that are well known to those skilled in the art.

The recombinant microbial host cell is cultivated in a suitable culture medium. The term "suitable culture medium" generally denotes a culture medium providing the nutrients that are essential for or beneficial to the maintenance and/or growth of said host cell, such as carbon sources; nitrogen sources such as ammonium sulfate; phosphorus sources, for example monobasic potassium phosphate; trace elements, for example copper, iodide, iron, magnesium, zinc or molybdate salts; vitamins and other growth factors such as amino acids or other growth promoters. An antifoam may be added if needed. According to the invention, this suitable culture medium may be chemically defined or complex. Notably, the culture medium may comprise a simple carbon source, such as glucose, fructose, xylose, ethanol, glycerol, galactose, sucrose, cellulose, cellobiose, starch, glucose polymers, molasses, or byproducts of these sugars.

In general, the conditions for cultivating the microorganisms according to the invention are readily adaptable by a person skilled in the art, as a function of the microorganism.

According to the invention, any cultivation method for the industrial-scale production of molecules of interest may be envisioned. Advantageously, the cultivation is performed in bioreactors, notably in batch, fed-batch, chemostat and/or continuous cultivation mode. The cultivation is generally performed in bioreactors, with possible solid and/or liquid preculturing steps in Erlenmeyer flasks, with a suitable culture medium.

Further aspects and advantages of the present invention will be described in the following examples, which should be regarded as illustrative and not limiting.

### EXAMPLES

### Material and methods

### General reagents, bacterial strains, plasmids and growth conditions.

Restriction enzymes (New England Biolabs), T4 DNA ligase (Promega), Phusion DNA polymerase (Thermo Scientific) were used for routine cloning methods. Bacterial strains and plasmids used in this study are listed below in Tables 1 and 2. For routine cloning, *E. coli* DH5α was used and maintained in Luria-Bertani (LB) medium at 37 °C. For carotenoid production, the recombinant *E. coli* DH5α strains were grown for 24 hours at 37 °C on a rotary shaker at 250 rpm followed by 24 hours at 30 °C with the same agitation. Chloramphenicol (25 µg/mL) and/or carbenicillin (100 µg/mL) were added as required.

**Table 1: List of plasmids**

| **Plasmid ID** | **Part description** | **Antibiotic** | **Source** |
|---|---|---|---|
| Level 0 Destination Vectors | | | |
| pBP | Universal Level 0 entry cloning vector | Cm | Moore et al, ACS Synth. Biol. 2016, 5, 10, 1059-1069 |

| Level 0 Plasmids with parts | | | |
|---|---|---|---|
| pL0-CRTMsa | Level 0 cloning vector, pBP with ORF *crtM* (domesticated) from S. *aureus* | Cm | This study |
| pL0-CRTNsa | Level 0 cloning vector, pBP with ORF *crtM* (domesticated) from S. *aureus* | Cm | This study |
| pL0-BEZAsn | Level 0 cloning vector, pBP with ORF *bezA* (domesticated) from *Streptomyces. niveus* NRRL 2449 | Cm | This study |
| pL0-Ybl-B | Level 0 cloning vector, pBP with ORF *crtYc* and *crtYd* (codon optimized) from *Brevibacterium linens* ATCC 9175 | Cm | This study |
| pL0-Lpm-E | Level 0 cloning vector, pBP with ORF *crtL-ε* (codon optimized) from *Prochlorococcus marinus* MED4 | Cm | This study |
| pL0-Lcc-E | Level 0 cloning vector, pBP with ORF *crtL-ε* (codon optimized) from *Cyanobium* sp. CACIAM 14 | Cm | This study |
| pL0-Lph-E | Level 0 cloning vector, pBP with ORF *crtL-ε* (codon optimized) from *Prochlorococcus* sp. HOT208 | Cm | This study |
| pL0-Lvl-E | Level 0 cloning vector, pBP with ORF *crtL-ε* (codon optimized) from *Vulcanococcus limneticus* | Cm | This study |
| pL0-Lsb-E | Level 0 cloning vector, pBP with ORF *crtL-ε* (codon optimized) from *Synechococcus* sp. BS55DK | Cm | This study |
| pL0-CCDlof | Level 0 cloning vector, pBP with ORF *ccd1* (codon optimized) from *Osmanthus fragrans* | Cm | This study |

| Level 1 Destination Vectors | | | |
|---|---|---|---|
| pTU1-A-RFP | Expression and cloning vector Level 1 position A with RFP as negative marker | Amp | Moore et al, ACS Synth. Biol. 2016, 5, 10, 1059-1069 |
| pTU1-B-RFP | Expression and cloning Level 1 position B with RFP as negative marker | Amp | Moore et al, ACS Synth. Biol. 2016, 5, 10, 1059-1069 |
| pTU1-C-RFP | Expression and cloning vector Level 1 position C with RFP as negative marker | Amp | Moore et al, ACS Synth. Biol. 2016, 5, 10, 1059-1069 |

| Level 1 Plasmids with TU | | | |
|---|---|---|---|
| pL1-A-M | Expression and cloning vector Level 1 position A, with *crtM* from pL0-CRTMsa with promoter SJM915, RBS TL9, terminator Bba_B0012 | Amp | This study |
| pL1-B-N | Expression and cloning vector Level 1 position B, with *crtN* from pL0-CRTNsa with promoter SJM915, RBS TL9, terminator Bba_B0012 | Amp | This study |
| pL1-A-BEZAsn-2 | Expression and cloning vector Level 1 position A with *bezAsn* from pL0-BEZAsn with promoter JH2300, RBS TL5, terminator Bba_B0012 | Amp | This study |
| pL1-B-Ybl-B | Expression and cloning vector Level 1 position B, with *crtYcYd* from pL0-YcYd with promoter SJM915, RBS TL9, terminator Bba_B0015 | Amp | This study |
| pL1-B-Lpm-E | Expression and cloning vector Level 1 position B, with *crtL-ε* from pL0-Lpm-Ewith promoter SJM915, RBS TL9, terminator Bba_B0015 | Amp | This study |
| pL1-B-Lcc-E | Expression and cloning vector Level 1 position B, with *crtL-ε* from pL0-Lcc-E with promoter SJM915, RBS TL9, terminator Bba_B0015 | Amp | This study |
| pL1-B-Lph-E | Expression and cloning vector Level 1 position B, with *crtL-ε* from pL0-Lph-E with promoter SJM915, RBS TL9, terminator Bba_B0015 | Amp | This study |
| pL1-B-Lvl-E | Expression and cloning vector Level 1 position B, with *crtL-ε* from pL0-Lvl-E with promoter SJM915, RBS TL9, terminator Bba_B0015 | Amp | This study |
| pL1-B-Lsb-E | Expression and cloning vector Level 1 position B, with *crtL-ε* from pL0-Lsb-E with promoter SJM915, RBS TL9, terminator Bba_B0015 | Amp | This study |
| pL1-C-CCDlof | Expression and cloning vector Level 1 position B, with *ccd1* from pL0-CCDlof with promoter JH2300, RBS TL5, terminator Bba_B0012 | Amp | This study |

| Level 2 Destination Vectors | | | |
|---|---|---|---|
| pTU2-b-RFP | Expression vector Level 2 position A with RFP as negativ marker to assemble 3 TUs | Cm | Moore et al, ACS Synth. Biol. 2016, 5, 10, 1059-1069 |
| pTU2-a-RFP | Expression vector Level 2 position A with RFP as negativ marker to assemble 2 TUs | Cm | Moore et al, ACS Synth. Biol. 2016, 5, 10, 1059-1069 |
| pTU2-a-kan-RFP | Expression vector Level 2 position A with RFP as negativ marker to assemble 2 TUs | Kan | This study |

| Level 2 Plasmids with TUs | | | |
|---|---|---|---|
| pL2-MN | pTU2-a with pL1-A-M and pL1-B-N | Cm | This study |
| pL2k-Bsn2-CYbl | pTU2-a-kan-RFP with pL1-A-BEZAsn-2 and pL1-B-Yb1-B | Kan | This study |
| pL2k-Bsn2-CLpm | pTU2-a-kan-RFP with pL1-A-BEZAsn-2 and pL1-B-Lpm-E | Kan | This study |
| pL2k-Bsn2-CLcc | pTU2-a-kan-RFP with pL1-A-BEZAsn-2 and pL1-B-Lcc-E | Kan | This study |
| pL2k-Bsn2-CLph | pTU2-a-kan-RFP with pL1-A-BEZAsn-2 and pL1-B-Lph-E | Kan | This study |
| pL2k-Bsn2-CLvl | pTU2-a-kan-RFP with pL1-A-BEZAsn-2 and pL1-B-Lv1-E | Kan | This study |
| pL2k-Bsn2-CLsb | pTU2-a-kan-RFP with pL1-A-BEZAsn-2 and pL1-B-Lsb-E | Kan | This study |

| Other plasmids | | | |
|---|---|---|---|
| pAC-LYCipi | Express *crtE, crtB, crtI* and *idi* from *Pantoea agglomerans* under tet promoter | Cm | Cunningham et al., Photosynth Res, 2007, 92, 245-259 |
| pAC-BETA | Express *crtE, crtB, crtI, idi* and *crtYpa* from *Pantoea agglomerans* under tet promoter | Cm | Cunningham et al., Photosynth Res, 2007, 92, 245-259 |

**Table 2: List of strains**

| **Strain** | **Relevant Characteristics** | **Source** |
|---|---|---|
| *E.coli* DH5α | *F- 80dlacZ M15* (*lacZYA-argF*) *U* 169 *recA1 endA* 1*hsdR*17(*rk-, mk+) phoAsupE44 -thi-*1 *gyr*A96 *relA*1 | Invitrogen |
| *S. aureus* | *Staphylococcus aureus* ATCC 23235 | ATCC |
| *S. niveus* NRRL 2449 | *Streptomyces niveus* NRRL 2449 /DSM 40292 | DSMZ |

### Bioinformatic analysis

Homologs of CrtL were identified using BLASTp search, using CrtL from *Prochlorococcus marinus* (WP_011132267) as query, respectively. Sequences with significant homology (sequence identity > 50%) were selected.

### Construction of expression plasmids using Ecoflex assembly

Plasmids pAC-LYCipi (Addgene_53279, containing *crtE, crtB, crtI,* and *idi* genes of *Pantoea agglomerans*) and pAC-BETA (Addgene_53272, containing *crtE, crtB, crtI,* and *crtY* carotenoid pathway genes of *Pantoea agglomerans*) were obtained from Addgene (Cunningham et al. Plant Cell. 6 (1994) 1107-1121). Expression plasmids used in this study were constructed on destination plasmid level 1 or 2, using described procedures of MoClo Ecoflex kit (Moore et al. ACS Synth. Biol. 5 (2016) 1059-1069) (Addgene kit#1000000080). These two plasmids have pSB1A2 backbone from iGEM registry and pMB1 derived from pUC-19 as replication origin. The procedure of Ecoflex assembly was used as described (Moore et al. *supra*): for level 0 assembly, 100 fmol of each DNA part was combined with 50 fmol of destination plasmid and incubated with *BsmB*I and T4 ligase for 25 cycles of 10 min at 37°C and 10 min at 16 °C, followed by 5 min at 50 °C and 10 min at 80 °C. For level 1 assembly, 50 fmol of each DNA part was combined with 50 fmol of destination plasmid and incubated with *Bsa*I and T4 ligase for 25 cycles of 10 min at 37 °C and 10 min at 16 °C, followed by 5 min at 50 °C and 10 min at 80 °C. For level 2 assembly, 20 fmol of each DNA part was combined with 20 fmol of destination plasmid and incubated with *BsmB*I and T4 ligase for 25 cycles of 10 min at 37 °C and 10 min at 16 °C, followed by 5 min at 50 °C and 10 min at 80 °C. One µL of Golden Gate reaction mix was transformed into 20 µL of chemically competent *E. coli* DH5α using heat shock transformation. After recovery in 200 µL of LB medium, 150 µL of cells were plated into LB plates with appropriate antibiotic and grown at 37°C overnight. Plasmid backbone pL2k, which was used for coexpression of *bezA* and lycopene cyclase genes, was generated from Ecoflex pTU2-a destination plasmid by changing the resistance to kanamycine using IVA cloning (García-Nafría et al., Sci. Rep. 6 (2016) 27459).

To obtain genes for cloning, *crtM* (encoding diapophytoene synthase, Uniprot: Q2FV5), *crtN* (encoding diapophytoene desaturase, Uniprot: Q2FV60) were PCR-amplified from genomic DNA of *Staphylococcus aureus* and the gene bezASN (encoding 6-GPP methyltransferase, SEQ ID NO: 17) was PCR-amplified from genomic DNA of *Streptomyces niveus* NRRL 2449. Other genes used in this study were codon optimized for *E. coli* using GeneArt^{®} software and synthetized by Twist Bioscience. Genes encoding lycopene cyclases used were *crtYc* (SEQ ID NO: 1) and *crtYd* (SEQ ID NO:2) that form CrtY_{BL}-β from *Brevibacterium linens,* CrtY_{PA}-β from *Pantoe agglomerans* (SEQ ID NO: 3), *crtL_{VL}-ε* from *Vulcanococcus limneticus* (SEQ ID NO:8), *crtL_{SB}-ε* from *Synechococcus* sp. BS55DK (SEQ ID NO:9), *crtL_{CC}-ε* from *Cyanobium* sp. CACIAM 14 (SEQ ID NO:10), *crtL_{PM}-ε* from *P. marinus* MED4 (SEQ ID NO:7) and *crtL_{PH}-ε* from *Prochlorococcus* sp. HOT208 (SEQ ID NO:11). Gene encoding the carotenoid cleavage dioxygenase was the gene encoding CCD1_{OF} from *Osmanthus fragrans* (SEQ ID NO: 50).

### Carotenoids production and extraction

*E. coli* DH5α harboring pAC-LYCipi was co-transformed with pL1-BezAsn-2, or pL2k-Bsn2-CXyz in order to produce methylated lycopene and methylated carotene, respectively. For production of acyclic methylated C30 carotenoids, *E. coli* DH5α cells were co-transformed with pL2-MN and pL1-BezA. Cells were pelleted by centrifugation (20°C, 4000 rpm) from 1 liter of culture, and carotenoids were repeatedly extracted from the pellets with 20 mL of HPLC grade acetone until all pigments were removed from the pellets. The colored supernatants were dried using a vacuum rotary evaporator. Samples were dissolved in 20 mL of 9:2 heptane/dichloromethane and loaded onto a silica gel column, which was pre-equilibrated with 9:2 heptane/dichloromethane. Carotenoids were eluted using the same solvent system. Each eluted sample was dried using a vacuum rotary evaporator and stored at -20°C under argon atmosphere until further use. Alternatively, crude acetone extracts of cell pellets were dried using a vacuum rotary evaporator and stored at -20°C under argon atmosphere, prior to be analyzed by mass spectrometry.

### In vitro CCD1 cleavage reactions

Extraction of carotenoids and CCD1_{OF} was performed as described with some modifications (Huang et al. J. Exp. Bot. 60 (2009) 3011-3022). A single colony of carotenoids-producing *E. coli* strains from a freshly-prepared plate was used to inoculate 50 mL LB medium containing appropriate antibiotics and grown at 30°C for *E. coli* DH5α cells harboring pL1-CCD1_{OF} that constitutively express CCD1_{OF} were grown in 50 mL LB with carbenicillin at 37°C overnight. Cells from the respective culture were harvested by centrifugation (5000 *g,* 40 min, 4 °C) and resuspended in 2 mL IX phosphate-buffered saline (140 mM NaCl, 4.3 mM Na₂HPO₄, 2.7 mM KCl, 1.47 mM K₂HPO₄, pH 7.3) containing 5 mM sodium ascorbate. Cells were lysed by sonification on salted ice four times for 30 s, using 28% and 10% of maximal amplitude for carotenoid and CCD1_{OF} extraction, respectively. One hundred *µ*L aliquot of each homogenized lysate were mixed together with 20 *µ*L of 20% (w/v) β-octylglucoside solution. The mixture was shaken vigorously and incubated at 30°C for 20 h in the dark. Reaction products were extracted once with equal volume of ethyl acetate (220 *µ*L). Resulting extracts were evaporated to dryness and resuspended in 50 *µ*L of LC-MS grade acetonitrile:isopropanol (1:1).

### LC-HR-ESI-MS analysis of carotenoids and apocarotenoids

Carotenoids eluted from the silica gel column or from the crude extracts were dissolved in 50 µL of LC-MS grade acetonitrile:isopropanol (1:1). One to 5 µL were injected onto a UPLC column (Luna Omega 1.6 µm Polar 100 Å, 150 x2.1 mm, Phenomenex) connected in the system (Ultimate 3000 RSLC, Thermo Scientific) that is coupled to a HR electrospray ionization quadrupole-time of flight (ESI-Q-TOF) mass spectrometer (MaXis II ETD, Bruker Daltonics). For carotenoids detection, the gradient of solvent A (water with 0.1% formic acid) and solvent B (acetonitrile with 0.08% formic acid) over a total runtime of 35 min (flow rate of 0.3 mL/min) was: isocratic elution at 80% B for 10 min, linear increase from 80% to 100% B over 1 min followed by 100% B for 24 min, then decrease to 80% in 5 min. For apocarotenoids separation, the gradient of solvent A (water with 0.1% formic acid) and solvent B (methanol with 0.08% formic acid) over a total runtime of 62 min (flow rate of 0.25 mL/min) was: linear increase from 20% to 65% B over 12 min followed by an isocratic elutation at 65%B for 10 min, then a linear increase to 100% B over 10 min, staying at 100% B for 2à min then decrease to 20% B in 4 min. In the first half minute of each run, a sodium formate solution was injected directly into the ion source as an internal reference for calibration. The mass range *m*/*z* from 100 to 800 in positive ion mode was acquired. The acquisition parameters of the ESI source were set up as follows: nebulize gas 2.4 bar, dry heater 200 °C, dry gas 8.0 L/min, capillary voltage 4500 V, end plate offset 500 V and charging voltage 2000 V. For LC-MS/MS, the auto MS/MS mode (collision energy 30.0 eV) was chosen with the same parameters as the MS method. The data were treated with Data Analysis 4.3 (Bruker Daltonics).

### Results

### Production of acyclic methylated carotenoids in E. coli

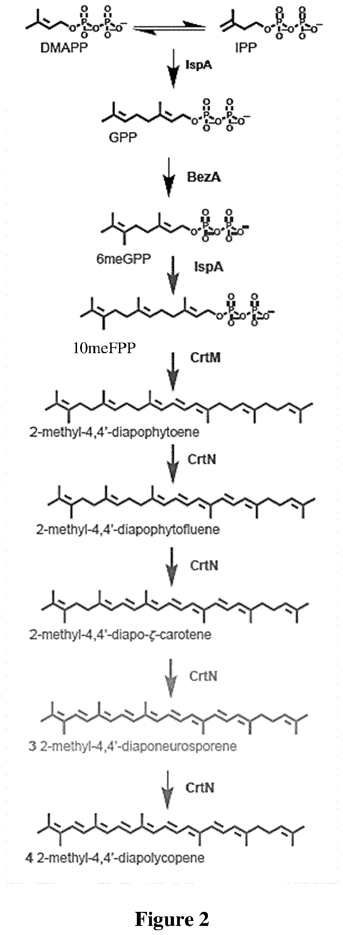

To demonstrate that the non-canonical C₁₁ building block, 6meGPP, generated by BezA can be accepted by downstream carotenoid enzymes, we initially used the C₃₀ pathway originating from bacteria as a model (Figure 2). The genes *crtM* and *crtN* from *Staphylococcus aureus* encoding the diapophytoene synthase and diapophytoene desaturase, respectively, were assembled on one plasmid, each under the control of SJM915 promoter and TL9 RBS (pL2-MN). The gene *bezA* from the benzastatin cluster in *Streptomyces niveus* was co-expressed in a separate plasmid under the control of the same promoter and RBS as in pL2-MN (pL1-BEZAsn-1).

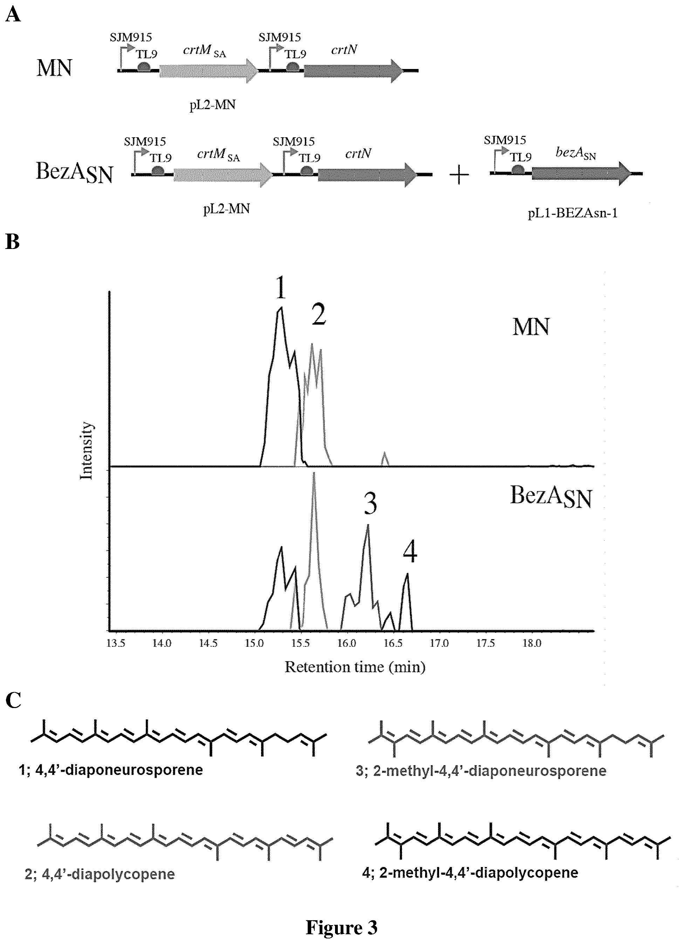

UHPLC (Ultra-high-pressure liquid chromatography) coupled to high resolution electrospray mass spectrometry (UHPLC-HR-ESI-MS) analysis of the acetone extracts of recombinant *E. coli* cells revealed two major new products of monomethylated C₃₁ carotenoids in cells expressing all three genes, 2 or 2'-methyl-4,4'-diaponeurosporene **(3,** C₃₁H₄₄, Rt = 10 min; M₊°_{obs}: *m*/*z* at 416.3435; M+°_{theoretical}: 416.3130) and 2 or 2'-methyl-4,4'-diapo-**ζ**-carotene (**4**; C₃₁H₄₆, Rt = 10.7 min; M₊°_{obs}: *m*/*z* at 418.3591; M₊°_{theoretical}: 418.3599) (Figure 3). Major natural C₃₀ products, 4,4'-diapolycopene **(1)** and 4,4'-diaponeurosporene (**2**), were also produced (Figure 3). Dimethylated products could be detected but with a very low yield. Our results also suggest that methylation influences the desaturation pattern of CrtN, shifting to products with less conjugated doubles. These experiments confirmed that 6meGPP can be incorporated into terpene pathways by downstream enzymes.

To increase the yield of C₃₁ carotenoids, *bezA* expression was optimized by screening five promoter-RBS combinations with different strength available in the Ecoflex MoClo kit ( Moore et al., ACS Synth. Biol. 5 (2016) 1059-1069). The strongest promoter-RBS pair tested (the resulting plasmid named pL1-BEZAsn-2), J23100-TL5, led to the highest production of mono-methylated 4,4'-diaponeurosporene **(3)** that was used as read-out. This suggests that GPP methylation is at least one of the rate-limiting steps.

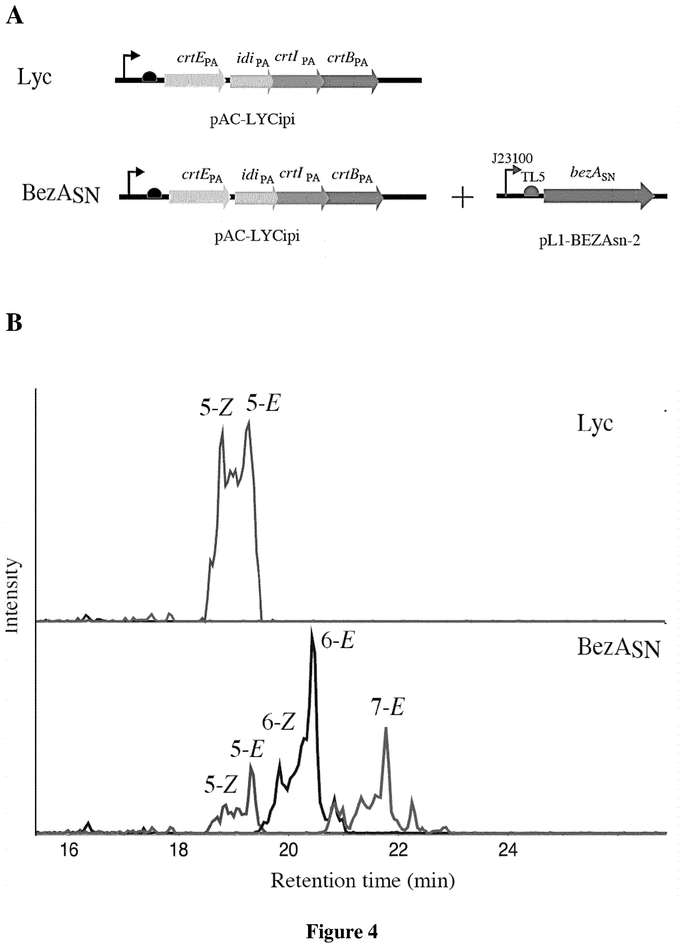

Next, we set out to co-express *bezA* encoded in pL1-BEZAsn-2 with *crtE, crtB* and *crtI* from *Pantoa agglomerans* of the C₄₀ pathway that were encoded in the plasmid pAC-LYCipi in *E. coli* (Cunningham et al. Plant Cell. 8 (1996) 1613-1626) (Figure 4). UHPLC-HR-ESI-MS analysis of acetone extracts of the recombinant strain confirmed the production of mono- and di-methylated lycopene: **6** (C₄₁H₅₈; Rt = 20.2 min; M₊°_{obs}: *m*/*z* at 550.4533; M₊°_{theoretical}: 550.4538) and **7** (C₄₂H₆₀; Rt = 20.9 min; M₊°_{obs}: *m*/*z* at 564.4690; M₊°_{theoretical}: 564.4695), in addition to the natural product lycopene **(5)** (Figure 4). Detailed tandem MS analysis allowed to distinguish Z or (*all-E*)-configuration in these products, based on characteristic fragment ions for each isomer. It is not possible to determine the position of the methyl group at this stage by tandem MS analysis, because the fragmentation pattern of lycopene is a series of fragments with a difference of 14 Da (-CH₂). Worth of note, related lycopenes were the only products accumulated in this system, suggesting that the number of desaturations catalyzed by CrtI is not impacted by methylation, contrary to CrtN in the C₃₀ pathway. Moreover, methylated lycopenes were present as major products, thereby facilitating downstream cyclization and cleavage steps towards irone production.

### Production of irone rings on the C₄₀-carotene backbone

Motivated by the *in vivo* production of methylated lycopene using *bezA,* the next step was to include a suitable lycopene cyclase into the pathway. Carotenoids with two *β*-ionone rings that are biosynthesized by lycopene *β*-cyclases are common in all carotenoid-producing organisms. A recent work has demonstrated that two bacterial lycopene *β*-cyclases, CrtY_{BL}-β from *Brevibacterium linens* and CrtY_{PA}-β from *Pantoe agglomerans,* were promiscuous and capable to form non-natural cyclic *β*-C₃₀ carotenoids (Kim et al. Sci. Rep. 6 (2016) 21987). Therefore, we co-expressed *bezA* together with *crtY_{BL}-β* or *crtY_{PA}-β* in one plasmid (pL2k-Bsn2-CXyz, Figure 5A). *E. coli* cells harboring the lycopene pathway and either co-expression plasmid produced cyclic C₄₁ and C₄₂ carotenes as well as the natural C₄₀ carotenes, as revealed by UHPLC-HR-ESI-MS analysis (Figure 5B). Each family of carotene products contained a mixture of isomers. Tandem MS analysis confirmed that these new products contain end cycles. Fragment ions with elimination of a xylene (C₈H₁₀, 106 Da) or a trimethylcyclohexene (C₇H₈, 92 Da) motif, which is characteristic of non-oxidized cyclized carotenoids (Neto et al., Mass Spectrom. 30 (2016) 1540-1548), were found for all new compounds. For monomethylated carotene **9,** it is not possible to determine the number and the position of the rings with accuracy. Nevertheless, tandem MS showed the presence of both fragments corresponding to ionone (Drummond et al. ACS Synth. Biol. 8 (2019) 1303-1313 ) and irone end-group (*m*/*z* 177.1638 and 191.1789, respectively), suggesting that both ends can be cyclized by CrtY_{BL/PA}-β. To provide unambiguous evidence that the methylated-end is cyclized, detailed tandem MS analysis was performed for cyclic C₄₂-carotenes **(10** or **13,** C₄₂H₅₆, M+obs: m/z at 564.4690; M+_{theoretical}: 564.4690 m/z). Only the fragment ion of irone end-group (m/z at 191.1789) but not that of ionone was detected (m/z at 177.1638), as compared to the C₄₀-carotene (Köksal et al. Biochemistry. 51 (2012) 3011-3020).

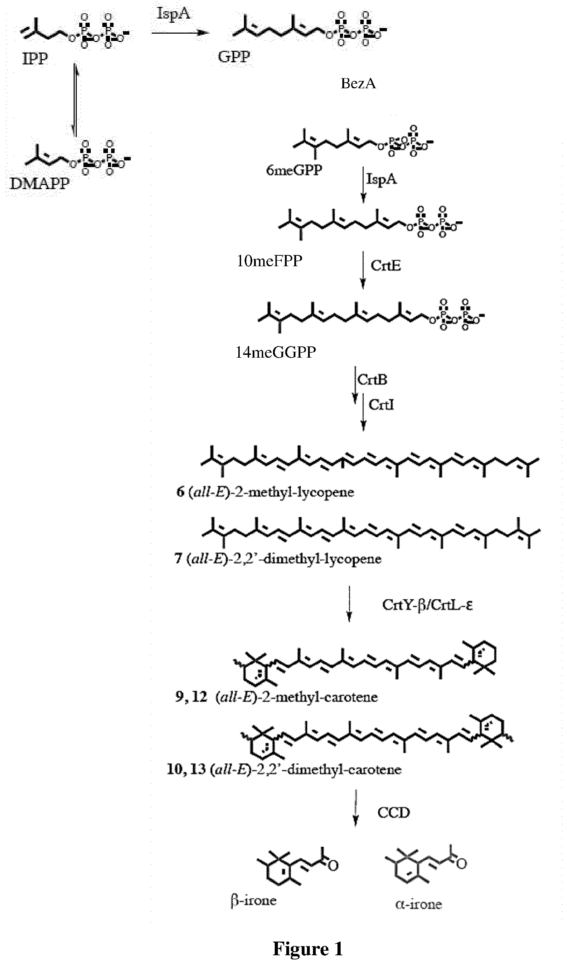

To generate α-irone motif (double bond between C4-C5) (Figure 1), it was necessary to use lycopene ε-cyclases. Carotenoids with *α*-ionone rings are found mainly in plants and in some cyanobacteria. CrtL lycopene cyclases from cyanobacteria are known to produce mainly the ε-carotene that consists of both *β* - and *α*-ionone rings (Stickforth et al. Arch. Microbiol. 179 (2003) 409-415). A characterized CrtL from *Prochlorococcus marinus* (CrtL_{PM}-ε) (Stickforth et al., *supra*) as well as four other homologues from *Vulcanococcus limneticus* (CrtL_{VL}-ε), *Synechococcus* sp. (CrtL_{SB}-ε), *Cyanobium* sp. (CrtL_{CC}-ε), and *Prochlorococcus* sp. (CrtL_{PH}-ε) were selected. Each corresponding gene was cloned into the co-expression plasmid of *bezA* (pL2k-Bsn2-CXyz). Similar to CrtY_{BL/PA}-β, all five cyanobacterial CrtLs were able to cyclize mono- and dimethyllycopene with various yields (Figure 5B). Tandem MS analysis of cyclic **10** or **13,** as described above, confirmed the presence of the irone ring motif. Among them, CrtL_{VL}-ε appeared to have a best production of methylated carotenes.

Given the specificity of the lycopene cyclase used, β- or α-irone motif should be likely formed in the new C42-based carotenes. To support this, tandem MS analysis of the cognate C40-carotene products **(8** or **11)** showed different characteristic fragment ions: *m*/*z* at 307.2421 for **8** (two β rings generated by CrtY-β) and 321.2565/388.3124 for **11** (one β- and one α-ring) generated by CrtL-ε (Figure S5B). Same fragment ions were observed in the C42-carotene produced by the same cyclase, indicating the same ring structure as in the natural C40-carotene product.

### Production of β- and α-irones by cleavage of 2, 2'-dimethyl-carotene

Carotenoid cleavage enzymes (CCDs) catalyze the formation of apocarotenoids by double-bond cleavage of cyclic carotenoids. This constitutes the last step of the designed irone pathway, which requires cleavage of the double bonds between C9-C10 and C9'-C10' of demethylated carotenes regardless of the configuration of the irone motif. Five distinct families of CCDs exist with different substrates and double-bond cleavage specificities (Ahrazem et al., Int. J. Mol. Sci. 2016, 17(11), 1781). Among them, the CCD1 enzyme has broad substrate specificity and cleaves at multiple double bond positions (Vogel et al., J. Biol. Chem. 283 (2008) 11364-11373).

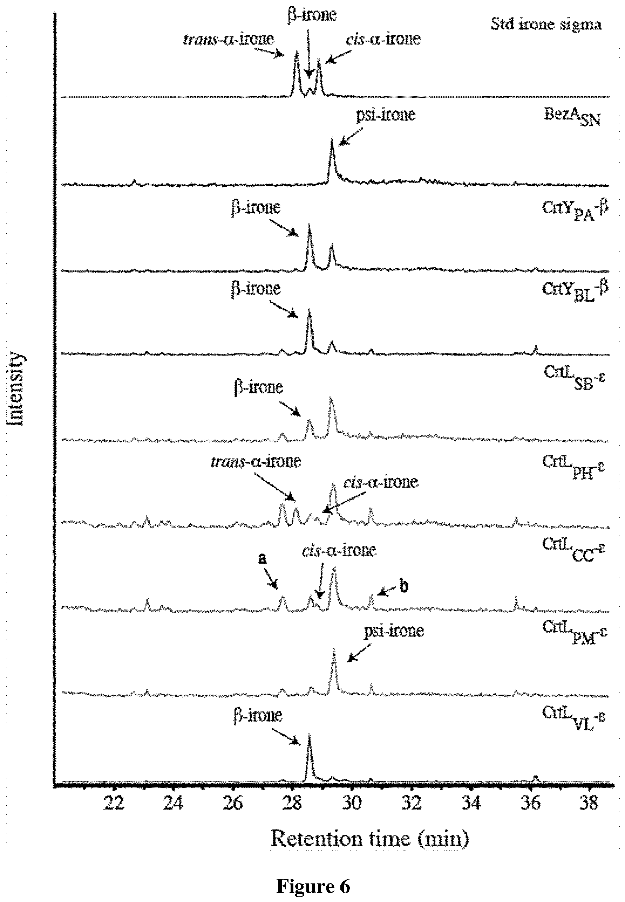

CCD1 from *Osmanthus fragrans* (CCD1_{OF}) was extracted in the presence of β-octylglucoside from the expressing *E. coli* strain without further purification. Crude extracts *of E. coli* strains producing irone bearing carotenes were mixed with CCD1_{OF} at 1:1 (v/v) and incubated overnight at 30°C. The reaction products were analyzed by UHPLC-HR-ESI-MS compared to the Irone Alpha standard. Depending of the cyclase, β*-, trans-* and/or *cis*-α-irone were observed only in reactions where cyclic carotenoids were present (*i.e*. extracts of cells expressing the lycopene cyclase), in addition to ionones (Figure 6). Specifically, the CrtY-β generated C42-carotenes yielded β-irone as the only product. On the contrary, the irone profiles varied with the used CrtL-ε. CrtL_{VL}-ε and CrtL_{SB}-ε led to only β-irone, whereas the other cyclases generated α-irones additionally. Interestingly, the α-irones produced in CrtL_{PH}-ε extracts consisted both *trans-* and *cis-*configuration, while only *cis-* ones were observed in CrtL_{CC}-ε extracts.

These experiments demonstrate the applicability of CCD1_{OF} in cleavage of nonnatural methylated-carotenes to generate irones. Furthermore, our data provide insight into the substrate specificity and activity of the tested CrtL-ε. They display a large discrepancy, thus cyanobacterial CrtL-ε represent a promising source to be exploited to generate specific irone motif.

We thus provide the first proof-of-principle of a functional, new-to-nature, irone biosynthetic pathway in *E. coli.* This pathway could serve as a versatile platform to produce specific irone isomers at will. We demonstrate here the production of *trans-,* and *cis*-α-irone as well as β-irone that has not been discovered in natural resources.

## Claims

1. A method of producing an irone compound comprising culturing a recombinant microbial host cell comprising a heterologous nucleic acid encoding a lycopene cyclase catalyzing the β- or ε-cyclization of one or both ends of 2-methyl-lycopene and/or the β- or ε-cyclization of one or both ends of 2,2'-dimethyl-lycopene, under conditions suitable to produce said irone compound and optionally recovering said irone compound.

2. The method of claim 1, wherein the lycopene cyclase is selected from the group consisting of wild-type cyanobacterial CrtL-type lycopene ε-cyclases, wild-type bacterial CrtY-type lycopene β-cyclases and wild-type heterodimeric-type lycopene cyclases of Gram positive bacteria, and variants thereof exhibiting lycopene cyclase activity and having at least 70% sequence identity to any of these cyclases.

3. The method of claim 1 or 2, wherein the lycopene cyclase comprises, or consists of, an amino acid sequence selected from the group consisting of SEQ ID NO: 10, 8, 9, 11, 1, 2 and 3 and variants thereof exhibiting lycopene cyclase activity and having at least 70 %, preferably at least 80%, more preferably at least 90%, 95%, 96%, 97%, 98% or 99%, sequence identity to any of SEQ ID NO: 10, 8, 9, 11, 1, 2 and 3.

4. The method of any one of claims 1 to 3, wherein the lycopene cyclase comprises, or consists of, an amino acid sequence selected from the group consisting of SEQ ID NO: 8 to 11, and variants thereof exhibiting lycopene cyclase activity and having at least 70 %, preferably at least 80%, more preferably at least 90%, 95%, 96%, 97%, 98% or 99%, sequence identity to any of SEQ ID NO: 8 to 11.

5. The method of any one of claims 1 to 3, wherein the lycopene cyclase comprises, or consists of, an amino acid sequence selected from the group consisting of SEQ ID NO: 10, and variants thereof exhibiting lycopene cyclase activity and having at least 70 %, preferably at least 80%, more preferably at least 90%, 95%, 96%, 97%, 98% or 99%, sequence identity to SEQ ID NO: 10.

6. The method of any one of claims 1 to 3, wherein the lycopene cyclase comprises, or consists of, an amino acid sequence selected from the group consisting of SEQ ID NO: 1 to 3, and variants thereof exhibiting lycopene β-cyclase activity and having at least 70 %, preferably at least 80%, more preferably at least 90%, 95%, 96%, 97%, 98% or 99%, sequence identity to any of SEQ ID NO: 1 to 3.

7. The method of any one of claims 1 to 6, wherein the recombinant microbial host cell further comprises a heterologous nucleic acid encoding a geranyl pyrophosphate (GPP) C6-methyltransferase.

8. The method of claim 7, wherein the GPP C6-methyltransferase comprises, or consist of, an amino acid sequence selected from the group consisting of SEQ ID NO: 17 to 31 and variants thereof exhibiting GPP C6-methyltransferase activity and having at least 70% sequence identity to SEQ ID NO: 17 to 31.

9. The method of any one of claims 1 to 8, wherein the recombinant microbial host cell further comprises a heterologous nucleic acid encoding a farnesyl diphosphate (FPP) synthase and/or a heterologous nucleic acid encoding a geranylgeranyl diphosphate (GGPP) synthase and/or a heterologous nucleic acid encoding a polypeptide exhibiting FPP synthase and GGPP synthase activities.

10. The method of any one of claims 1 to 9, wherein the recombinant microbial host cell further comprises a heterologous nucleic acid encoding a phytoene synthase and a heterologous nucleic acid encoding a lycopene-forming phytoene desaturase.

11. The method of any one of claims 1 to 10, wherein the recombinant microbial host cell further comprises a heterologous nucleic acid encoding a carotenoid cleavage dioxygenase.

12. The method of any one of claims 1 to 11, wherein the recombinant microbial host has been genetically modified to reduce the activity of an endogenous PgpB phosphatase, an endogenous CpdB phosphatase and/or an endogenous MetJ repressor by comparison to the non-modified microbial host cell.

13. The method of any one of claims 1 to 12, wherein the recombinant microbial host is a bacterium or a yeast.

14. The recombinant microbial host as defined in any one of claims 1 to 13.

15. Use of a recombinant microbial host cell according to claim 14 to produce an irone compound.
